# EUROPEAN PATENT SPECIFICATION

(11) **EP 1 731 602 B1**
(45) Date of publication and mention of the grant of the patent: **31.03.2010**
(21) Application number: 04772797.9
(22) Date of filing: 03.09.2004
(51) Int. Cl.: C07K 14/605

(54) **PHARMACEUTICAL COMPOSITION FOR LOWERING BLOOD SUGAR LEVEL**
PHARMAZEUTISCHE ZUSAMMENSETZUNG ZUR HERABSETZUNG DES BLUTZUCKERSPIEGELS
COMPOSITION PHARMACEUTIQUE POUR DIMINUER LE TAUX DE SUCRE DANS LE SANG

(30) Priority: 01.03.2004 JP 2004056452; 20.08.2004 JP 2004240607
(43) Date of publication of application: 13.12.2006
(73) Proprietor: PHARMAFRONTIER CO., LTD., Kyoto-shi, Kyoto (JP)
(72) Inventor: HIRASAWA, Akira, Uji-shi, Kyoto (JP); TSUJIMOTO, Gozo, Tokyo 1580091 (JP)
(74) Representative: De Clercq, Ann G. Y.
(86) International application number: PCT/JP2004/012848
(87) International publication number: WO 2005/083070

(56) References cited:
- WO-A1-03/068959
- WO-A1-2004/004000
- WO-A1-2004/065960
- WO-A2-00/00611
- WO-A2-00/50596
- WO-A2-02/067868
- DATABASE EPO Proteins [Online] 3 March 2003 (2003-03-03), "Sequence 1466 from Patent EP1270724." XP002424928 retrieved from EBI accession no. EPOP:AX647274 Database accession no. AX647274
- DATABASE Geneseq [Online] 20 December 2000 (2000-12-20), "A human G-protein coupled receptor designated 14273." XP002424929 retrieved from EBI accession no. GSP:AAB08538 Database accession no. AAB08538
- DATABASE USPTO Proteins [Online] 12 September 2003 (2003-09-12), "Sequence 4 from patent US 6395877." XP002424930 retrieved from EBI accession no. USPOP:AAQ81190 Database accession no. AAQ81190
- ADEYEYE E I ET AL: "Fatty acid composition of six varieties of dehulled African yam bean (Sphenostylis stenocarpa) flour" INTERNATIONAL JOURNAL OF FOOD SCIENCES AND NUTRITION, vol. 50, no. 5, September 1999 (1999-09), pages 357-365, XP009080714 ISSN: 0963-7486
- HIRASAWA AKIRA ET AL: "Free fatty acids regulate gut incretin glucagon-like peptide-1 secretion through GPR120" NATURE MEDICINE, NATURE PUBLISHING GROUP, NEW YORK, NY, US, vol. 11, no. 1, January 2005 (2005-01), pages 90-94, XP002410251 ISSN: 1078-8956
- FREDRIKSSON R. ET AL: 'Seven evolutionarily conserved human rhodopsin, G protein-coupled receptors lacking close relatives.' FEBS LETT. vol. 554, no. 3, 20 November 2003, pages 381 - 388, XP004472955
- 'Kagaku Dai Jiten Henshu Iinkai, Kagaku Dai Jiten 8, reeduced-size edition.', 1962 page 552, XP002991239
- 'Seikagaku Jiten.', 1990 XP002991240
- MICHINORI OOKI.: 'Kagaku Dai Jiten.', 1989 XP002991241
- HOLZ G.G. ET AL: 'Glucagon-like peptide-1 synthetic analogs: new therapeutic agents for use in the treatment of diabetes mellitus.' CURR. MED.CHEM. vol. 10, no. 22, 2003, pages 2471 - 2483, XP009035149

### SEQUENCE LISTING

<110> PHARMAFRONTIER CO., LTD.
<120> Pharmaceutical composition for lowering blood sugar level
<130> GOZO-001-EP
<140> EP 04772797.9
   <141> 2004-09-03
<150> JP2004056452
   <151> 2004-03-01
<150> JP2004240607
   <151> 2004-08-20
<160> 7
<170> PatentIn version 3.1
<210> 1
   <211> 377
   <212> PRT
   <213> Homo sapiens
<400> 1
   Met Ser Pro Glu Cys Ala Arg Ala Ala Gly Asp Ala Pro Leu Arg Ser
   1 5 10 15
   Leu Glu Gln Ala Asn Arg Thr Arg Phe Pro Phe Phe Ser Asp Val Lys
   20 25 30
   Gly Asp His Arg Leu Val Leu Ala Ala Val Glu Thr Thr Val Leu Val
   35 40 45
   Leu Ile Phe Ala Val Ser Leu Leu Gly Asn Val Cys Ala Leu Val Leu
   50 55 60
   Val Ala Arg Arg Arg Arg Arg Gly Ala Thr Ala Cys Leu Val Leu Asn
   65 70 75 80
   Leu Phe Cys Ala Asp Leu Leu Phe Ile Ser Ala Ile Pro Leu Val Leu
   85 90 95
   Ala Val Arg Trp Thr Glu Ala Trp Leu Leu Gly Pro Val Ala Cys His
   100 105 110
   Leu Leu Phe Tyr Val Met Thr Leu Ser Gly Ser Val Thr Ile Leu Thr
   115 120 125
   Leu Ala Ala Val Ser Leu Glu Arg Met Val Cys Ile Val His Leu Gln
   130 135 140
   Arg Gly Val Arg Gly Pro Gly Arg Arg Ala Arg Ala Val Leu Leu Ala
   145 150 155 160
   Leu Ile Trp Gly Tyr Ser Ala Val Ala Ala Leu Pro Leu Cys Val Phe
   165 170 175
   Phe Arg Val Val Pro Gln Arg Leu Pro Gly Ala Asp Gln Glu Ile Ser
   180 185 190
   Ile Cys Thr Leu Ile Trp Pro Thr Ile Pro Gly Glu Ile Ser Trp Asp
   195 200 205
   Val Ser Phe Val Thr Leu Asn Phe Leu Val Pro Gly Leu Val Ile Val
   210 215 220
   Ile Ser Tyr Ser Lys Ile Leu Gln Thr Ser Glu His Leu Leu Asp Ala
   225 230 235 240
   Arg Ala Val Val Thr His Ser Glu Ile Thr Lys Ala Ser Arg Lys Arg
   245 250 255
   Leu Thr Val Ser Leu Ala Tyr Ser Glu Ser His Gln Ile Arg Val Ser
   260 265 270
   Gln Gln Asp Phe Arg Leu Phe Arg Thr Leu Phe Leu Leu Met Val Ser
   275 280 285
   Phe Phe Ile Met Trp Ser Pro Ile Ile Ile Thr Ile Leu Leu Ile Leu
   290 295 300
   Ile Gln Asn Phe Lys Gln Asp Leu Val Ile Trp Pro Ser Leu Phe Phe
   305 310 315 320
   Trp Val Val Ala Phe Thr Phe Ala Asn Ser Ala Leu Asn Pro Ile Leu
   325 330 335
   Tyr Asn Met Thr Leu Cys Arg Asn Glu Trp Lys Lys Ile Phe Cys Cys
   340 345 350
   Phe Trp Phe Pro Glu Lys Gly Ala Ile Leu Thr Asp Thr Ser Val Lys
   355 360 365
   Arg Asn Asp Leu Ser Ile Ile Ser Gly
   370 375
<210> 2
   <211> 361
   <212> PRT
   <213> Mus musculus
<400> 2
<210> 3
   <211> 22
   <212> DNA
   <213> Artificial Sequence
<220>
   <223> Description of Artificial Sequence:synthetic DNA
<400> 3 22
   atgtcccctg aatgcgcgcg gg 22
<210> 4
   <211> 22
   <212> DNA
   <213> Artificial Sequence
<220>
   <223> Description of Artificial Sequence:synthetic DNA
<400> 4
   gccagaaata atcgacaagt ca 22
<210> 5
   <211> 23
   <212> DNA
   <213> Artificial Sequence
<220>
   <223> Description of Artificial Sequence:synthetic DNA
<400> 5
   cgcacccgct ttcccttctt ctc 23
<210> 6
   <211> 25
   <212> DNA
   <213> Artificial Sequence
<220>
   <223> Description of Artificial sequence:synthetic DNA
<400> 6
   agctctttcc ttgatgcctt tgtga 25
<210> 7
   <211> 1389
   <212> DNA
   <213> Mus musculus
<400> 7
   cagatgagcg ctctctcaga cagcggcggg cggccgggcg cccggcatgt cccctgagtg      60
   tgcacagacg acgggccctg gcccctcgca caccctggac caagtcaatc gcacccactt     120
   ccctttcttc tcggatgtca agggcgacca ccggttggtg ttgagcgtcg tggagaccac     180
   cgttctgggg ctcatctttg tcgtctcact gctgggcaac gtgtgtgctc tagtgctggt     240
   ggcgcgccgt cggcgccgtg gggcgacagc cagcctggtg ctcaacctct tctgcgcgga      300
   tttgctcttc accagcgcca tccctctagt gctcgtcgtg cgctggactg aggcctggct     360
   gttggggccc gtcgtctgcc acctgctctt ctacgtgatg acaatgagcg gcagcgtcac     420
   gatcctcaca ctggccgcgg tcagcctgga gcgcatggtg tgcatcgtgc gcctccggcg     480
   cggcttgagc ggcccggggc ggcggactca ggcggcactg ctggctttca tatggggtta     540
   ctcggcgctc gccgcgctgc ccctctgcat cttgttccgc gtggtcccgc agcgccttcc     600
   cggcggggac caggaaattc cgatttgcac attggattgg cccaaccgca taggagaaat     660
   ctcatgggat gtgttttttg tgactttgaa cttcctggtg ccgggactgg tcattgtgat      720
   cagttactcc aaaattttac agatcacgaa agcatcgcgg aagaggctta cgctgagctt     780
   ggcatactct gagagccacc agatccgagt gtcccaacaa gactaccgac tcttccgcac     840
   gctcttcctg ctcatggttt ccttcttcat catgtggagt cccatcatca tcaccatcct     900
   cctcatcttg atccaaaact tccggcagga cctggtcatc tggccatccc ttttcttctg     960
   ggtggtggcc ttcacgtttg ccaactctgc cctaaacccc atactgtaca acatgtcgct    1020
   gttcaggaac gaatggagga agattttttg ctgcttcttt tttccagaga agggagccat    1080
   ttttacagat acgtctgtca ggcgaaatga cttgtctgtt atttccagct aactagcctc    1140
   tggtgccagg tgaaccacgg tgtgcatgta aagggagtta acttcaagga aagcccacca    1200
   gtgcgccctg ctttaaaaat acccgacttc caacagcagg catctacgga gccagcaaat    1260
   taaggaatga tcgctcagta taaaaatatt tttccttaaa agaactttct atgggttcct    1320
   tttgtgaact ttttttagtg tgtttgtaat atgatctagt taataaattt ttatttataa     1380
   ctgttccta                                                            1389

### SEQUENCE LISTING

<110> PHARMAFRONTIER CO., LTD.
<120> Pharmaceutical composition for lowering blood sugar level
<130> GOZO-001-EP
<140> EP 04772797.9
   <141> 2004-09-03
<150> JP2004056452
   <151> 2004-03-01
<150> JP2004240607
   <151> 2004-08-20
<160> 7
<170> PatentIn version 3.1
<210> 1
   <211> 377
   <212> PRT
   <213> Homo sapiens
<400> 1
   Met Ser Pro Glu Cys Ala Arg Ala Ala Gly Asp Ala Pro Leu Arg Ser
   1               5                   10                  15
   Leu Glu Gln Ala Asn Arg Thr Arg Phe Pro Phe Phe Ser Asp Val Lys
               20                  25                  30
   Gly Asp His Arg Leu Val Leu Ala Ala Val Glu Thr Thr Val Leu Val
           35                  40                  45
   Leu Ile Phe Ala Val Ser Leu Leu Gly Asn Val Cys Ala Leu Val Leu
       50                  55                  60
   Val Ala Arg Arg Arg Arg Arg Gly Ala Thr Ala Cys Leu Val Leu Asn
   65                  70                  75                  80
   Leu Phe Cys Ala Asp Leu Leu Phe Ile Ser Ala Ile Pro Leu Val Leu
                   85                  90                  95
   Ala Val Arg Trp Thr Glu Ala Trp Leu Leu Gly Pro Val Ala Cys His
               100                 105                 110
   Leu Leu Phe Tyr Val Met Thr Leu Ser Gly Ser Val Thr Ile Leu Thr
           115                 120                 125
   Leu Ala Ala Val Ser Leu Glu Arg Met Val Cys Ile Val His Leu Gln
       130                 135                 140
   Arg Gly Val Arg Gly Pro Gly Arg Arg Ala Arg Ala Val Leu Leu Ala
   145                 150                 155                 160
   Leu Ile Trp Gly Tyr Ser Ala Val Ala Ala Leu Pro Leu Cys Val Phe
                   165                 170                 175
   Phe Arg Val Val Pro Gln Arg Leu Pro Gly Ala Asp Gln Glu Ile Ser
               180                 185                 190
   Ile Cys Thr Leu Ile Trp Pro Thr Ile Pro Gly Glu Ile Ser Trp Asp
   195 200 205
   Val Ser Phe Val Thr Leu Asn Phe Leu Val Pro Gly Leu Val Ile Val
   210 215 220
   Ile Ser Tyr Ser Lys Ile Leu Gln Thr Ser Glu His Leu Leu Asp Ala
   225 230 235 240
   Arg Ala Val Val Thr His Ser Glu Ile Thr Lys Ala Ser Arg Lys Arg
   245 250 255
   Leu Thr Val Ser Leu Ala Tyr Ser Glu Ser His Gln Ile Arg Val Ser
   260 265 270
   Gln Gln Asp Phe Arg Leu Phe Arg Thr Leu Phe Leu Leu Met Val Ser
   275 280 285
   Phe Phe Ile Met Trp Ser Pro Ile Ile Ile Thr Ile Leu Leu Ile Leu
   290 295 300
   Ile Gln Asn Phe Lys Gln Asp Leu Val Ile Trp Pro Ser Leu Phe Phe
   305 310 315 320
   Trp Val Val Ala Phe Thr Phe Ala Asn Ser Ala Leu Asn Pro Ile Leu
   325 330 335
   Tyr Asn Met Thr Leu Cys Arg Asn Glu Trp Lys Lys Ile Phe Cys Cys
   340 345 350
   Phe Trp Phe Pro Glu Lys Gly Ala Ile Leu Thr Asp Thr Ser Val Lys
   355 360 365
   Arg Asn Asp Leu Ser Ile Ile Ser Gly
   370 375
<210> 2
   <211> 361
   <212> PRT
   <213> Mus musculus
<400> 2
<210> 3
   <211> 22
   <212> DNA
   <213> Artificial Sequence
<220>
   <223> Description of Artificial sequence:synthetic DNA
<400> 3
   atgtcccctg aatgcgcgcg gg 22
<210> 4
   <211> 22
   <212> DNA
   <213> Artificial sequence
<220>
   <223> Description of Artificial Sequence:synthetic DNA
<400> 4
   gccagaaata atcgacaagt ca 22
<210> 5
   <211> 23
   <212> DNA
   <213> Artificial Sequence
<220>
   <223> Description of Artificial Sequence:synthetic DNA
<400> 5
   cgcacccgct ttcccttctt ctc 23
<210> 6
   <211> 25
   <212> DNA
   <213> Artificial Sequence
<220>
   <223> Description of Artificial Sequence:Synthetic DNA
<400> 6
   agctctttcc ttgatgcctt tgtga 25
<210> 7
   <211> 1389
   <212> DNA
   <213> Mus musculus
<400> 7
   cagatgagcg ctctctcaga cagcggcggg cggccgggcg cccggcatgt cccctgagtg     60
   tgcacagacg acgggccctg gcccctcgca caccctggac caagtcaatc gcacccactt    120
   ccctttcttc tcggatgtca agggcgacca ccggttggtg ttgagcgtcg tggagaccac    180
   cgttctgggg ctcatctttg tcgtctcact gctgggcaac gtgtgtgctc tagtgctggt    240
   ggcgcgccgt cggcgccgtg gggcgacagc cagcctggtg ctcaacctct tctgcgcgga    300
   tttgctcttc accagcgcca tccctctagt gctcgtcgtg cgctggactg aggcctggct    360
   gttggggccc gtcgtctgcc acctgctctt ctacgtgatg acaatgagcg gcagcgtcac    420
   gatcctcaca ctggccgcgg tcagcctgga gcgcatggtg tgcatcgtgc gcctccggcg    480
   cggcttgagc ggcccggggc ggcggactca ggcggcactg ctggctttca tatggggtta    540
   ctcggcgctc gccgcgctgc ccctctgcat cttgttccgc gtggtcccgc agcgccttcc    600
   cggcggggac caggaaattc cgatttgcac attggattgg cccaaccgca taggagaaat    660
   ctcatgggat gtgttttttg tgactttgaa cttcctggtg ccgggactgg tcattgtgat    720
   cagttactcc aaaattttac agatcacgaa agcatcgcgg aagaggctta cgctgagctt    780
   ggcatactct gagagccacc agatccgagt gtcccaacaa gactaccgac tcttccgcac    840
   gctcttcctg ctcatggttt ccttcttcat catgtggagt cccatcatca tcaccatcct    900
   cctcatcttg atccaaaact tccggcagga cctggtcatc tggccatccc ttttcttctg    960
   ggtggtggcc ttcacgtttg ccaactctgc cctaaacccc atactgtaca acatgtcgct   1020
   gttcaggaac gaatggagga agattttttg ctgcttcttt tttccagaga agggagccat   1080
   ttttacagat acgtctgtca ggcgaaatga cttgtctgtt atttccagct aactagcctc   1140
   tggtgccagg tgaaccacgg tgtgcatgta aagggagtta acttcaagga aagcccacca   1200
   gtgcgccctg ctttaaaaat acccgacttc caacagcagg catctacgga gccagcaaat   1260
   taaggaatga tcgctcagta taaaaatatt tttccttaaa agaactttct atgggttcct   1320
   tttgtgaact ttttttagtg tgtttgtaat atgatctagt taataaattt ttatttataa   1380
   ctgttccta                                                           1389

## Description

### Technical Field

The present invention relates to a pharmaceutical composition for lowering blood sugar level and preventing or treating diabetes.

In addition, the present invention relates to a pharmaceutical composition for treating eating disorders.

Further, the present invention relates to a dietary supplement composition used for rational dieting.

### Background Art

The number of diabetic patients is markedly increased in society today, and social and economical costs considered necessary for the treatment thereof have reached a large amount of money, which provides a serious problem in the medical field desired to be early solved.

Diabetes does not enable glucose in blood to be normally metabolized, makes a so-called hyperglycemic state persist, and has various negative effects on the function of the body. Major complications of diabetes include retinopathy, nephropathy, neuropathy, and arteriosclerosis, and these complications have a risk causing serious pathologic conditions such as blindness, uremia, stroke, and cardiac infarction.

As a method for treating diabetes, the injection of insulin necessary for glucose metabolism is conducted in addition to dietetic therapy, ergotherapy, or the like to normalize metabolic function.

In addition to the method involving the direct administrationof insulin, researches, forexample, onamethod for promoting insulin production, insulin secretion, or the like to attain the lowering of blood sugar level are also actively carried out.

GLP-1 (glucagon-like peptide-1) is getting a lot of attention as a peptide hormone regulating insulin secretion. GLP-1 is a peptide hormone arising from the post-translation processing of proglucagon produced in L cells which are enteroendocrine cells present in the ileum, large intestine, or the like (Drucker, 1998). Since it induces insulin secretion in response to a glucose concentration, the peptide has been pointed out to have a possibility of exerting a therapeutic effect particularly against type II (non-insulin-dependent) diabetes (Drucker, 2001; Thorens and Waeber, 1993) . In addition, GLP-1 has been reported to have, for example, the effect of inducing the proliferation of β-cells or the neogenesis of β-cells from stem cells, and is probably effective in retarding the apoptosis of β-cells in type II diabetes and in maintaining the effect of pancreatic islet transplantation against type I diabetes.

In addition to the effect of regulating insulin secretion, GLP-1 has been shown to have the effect of inducing the stimulation of "feeling of satiety" and the reduction of eating by acting on the hypothalamus in the central nervous system (Turton, et al., 1996; Flint, et al., 1998); it is expected to have the therapeutic effects not only against diabetes but also against eating disorders including obesity. Recent studies have shown that substances such as fatty acid and TNF released from fat cells disturb insulin action, and a mechanism through which the elevation of blood sugar level due to obesity is promoted is also being elucidated. Thus, GLP-1 not only promoting insulin secretion but also simultaneously having the effect of suppressing obesity will provide a breakthrough therapeutic agent for treating diabetes.

In applying GLP-1 to the treatment of diabetes, however, some trouble has been caused e.g., by the points that the half-life of GLP-1 is short in the living body (Drucker, 2001; Thorens and Waeber, 1993) and that a method by injection has to be adopted in the administration thereof because it is peptidic.

A possible method for overcoming these problems is to regulate insulin secretion by promoting the secretion of endogenous GLP-1.

It has been previously pointed out that fatty acid is involved in the promotion of GLP-1 secretion. For example, it has been reported that giving a monounsaturated fatty acid to rats stimulates the secretion of GLP-1 (non-patent document 1) or that oleic acid or palmitic acid promotes the secretion of GLP-1 from the human intestinal cell line NCI-H716 (non-patent document 2). Meanwhile, however, there have been reported some results inconsistent with the above-mentioned reports, including that a polyunsaturated fatty acid or monounsaturated fatty acid did not affect insulin secretion via GLP-1 (non-patent document 3) and that the injection of a short-chain fatty acid (SCFA) into blood produced no change in the concentration of GLP-1 in plasma (non-patent document 4). In the case of the reported promotion of GLP-1 secretion by certain types of fatty acids, further, no mechanismof action thereof has been elucidated, which has also brought into question the validity of relation between fatty acid and GLP-1 promotion.

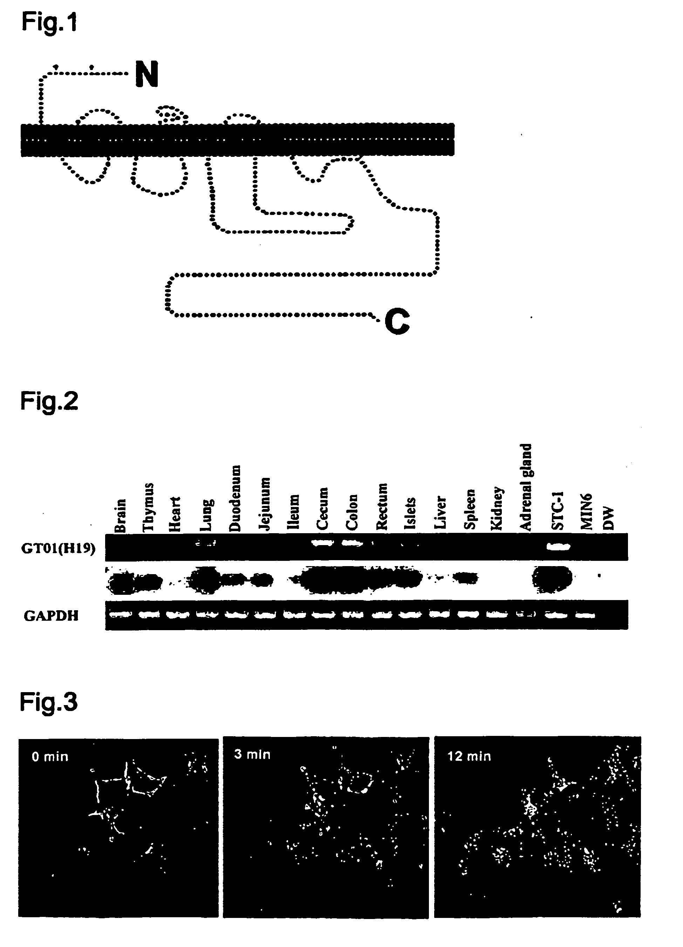

The composition according to the invention contains a GT01 protein as a G-protein coupled receptor and a ligand specifically binding to the GT01 protein. The G-protein coupled receptor is called a 7-pass transmembrane receptor (7TMR) because it has 7 transmembrane regions (see Figure 1), and involved in intracellular signaling through the activation of a coupled guanine nucleotide-binding protein (hereinafter referred to as G protein).

The G-protein coupled receptor is present on the surface of each functional cell in the living body, forms a target for a ligand molecule regulating the function, and conveys a signal into the cell through the binding with the ligand molecule. The cell receiving the conveyed signal is subjected to the activation or inhibition of the cellular function, resulting in the initiationof various in vivo reactions. Thus, elucidating the function of the G-protein coupled receptor has become highly important also in view of developing a medicine well regulating an in vivo reaction.

A vast amount of information of genome and cDNA has become available in recent years, and many G-protein coupled receptors have been identified; however, in many of those, the functions thereof and specific ligands therefor have not yet been demonstrated, the progress of analysis thereof having been awaited.

Human GT01 protein is called GPR120, and identical to the amino acid sequence deposited as NP_859529 in GenBank. However, no function of GPR120 has been addressed, and the biological role thereof is uncertain (non-patent document 5) .

In addition, it has 95% amino acid identity to a 14273 receptor as a G-protein coupled receptor. However, no ligand for the 14273 receptor has been identified, and the mechanism of action thereof has not been elucidated in detail. The 14273 receptor has been also observed to be expressed in the heart, and identified as an involvement in heart disease from the analysis of transgenic mice using a gene encoding the receptor. Thus, although the GT01 polypeptide disclosed in the invention and the 14273 receptor have a high amino acid sequence identity with each other, from the comparison of the functions thereof (to be described), it seems that they have the possibility of assuming physiologically completely different roles (see patent document 1 or 2).

The present inventors have already found that the GT01 protein according to the invention, together with a ligand therefor, acts to promote the secretion of CCK functioning in the control of eating (Japanese Patent Application No. 2003-180375). The present invention has additionally found that the GT01 protein, together with an agonist therefor, acts to promote the secretion of GLP-1 functioning in the lowering of blood sugar level and further the secretion of insulin. Thus, it is expected that the GT01 protein according to the invention, together with a ligand therefor, prevents or alleviates obesity by enabling the control of eating and the lowering of blood sugar level and also exerts an effect in the treatment of diabetes by producing the lowering of blood sugar level.
Patent document 1 : U.S. Patent No. 6,448,005B1 (entire text).
Patent document 2: National Publication of International Patent Application No. 2002-536997 (entire text).
Non-patent document 1: Rocca, A.S. et al., Endocrinology 142:1148-1155, 2001.
Non-patent document 2: Reimer, R.A. et al., Endocrinoloby 142:4522-4528, 2001.
Non-patent document 3: Cuche, G. et al., Am J Physiol Gastrointest Liver Physiol. 279: G925-930, 2000.
Non-patent document 4: Brynes, A.E., Am J Clin Nutr 72: 1111-1118, 2000.
Non-patent document 5: Fredriksson, R. et al., FEBS 554: 381-388, 2003.

### Disclosure of the Invention

Then, an object of the present invention is to provide a pharmaceutical composition for inducing the secretion of endogenous GLP-1 useful in treating type I or type II diabetes by promoting insulin secretion.

### Effect of the Invention

The pharmaceutical composition provided by the invention can be used to promote GLP-1 secretion and insulin secretion from cells. As a result, there can be expected the effects of the promoting of insulin secretion from pancreatic β-cells, the propagating of β-cells, the inducing of the neogenesis of β-cells from stem cells, or the like.

In addition, there can be expected the effects of the retarding of the apoptosis of β-cells in type II diabetes and the maintaining of the effect of pancreatic islet transplantation against type I diabetes.

Further, the use of a pharmaceutical composition containing a free fatty acid as a ligand for the GT01 polypeptide first demonstrated by the invention enables the regulation of CCK release from intestinal cells expressing the GT01 polypeptide. As a result, the regulation of a peripheral or central eating-control mechanism responsive to CCK is made possible, and the improvement of eating disorders and symptoms of diseases etc. associated therewith can be achieved.

Further, the accomplishment of rational dieting or the effect of enhancing appetite can be expected by the ingestion of a dietary supplement agent containing a free fatty acid as a ligand for the GT01 polypeptide of the invention.

Since the pharmaceutical composition provided by the invention is used to produce "feeling of satiety" to enable the reduction of eating to be induced, it also suppresses obesity probably facilitating diabetic symptoms and can be expected to be further effective in the treatment of diabetes via the lowering of blood sugar level.

Further, the method for determining a ligand or method for screening a substance altering the binding properties of a ligand to GT01, provided by the invention is used to enable the identification of a new compound capable of effectively controlling the secretion of GLP-1; the development of a therapeutic agent for diabetes via the lowering of blood sugar level can be expected.

Further, the screening method or screening kit provided by the invention is used to enable the identification of a new compound capable of regulating the binding mode of an existing ligand toGT01; the development of a therapeutic agent for diabetes via the lowering of blood sugar level can be expected.

### Best Mode for Carrying Out the Invention

### 1. Cloning of a gene encoding a GT01 polypeptide

For the purpose of the invention, "GT01 polypeptide" refers to a G-protein coupled receptor having the amino acid sequence represented by SEQ ID NO: 1 or SEQ ID NO: 2, or a G-protein coupled receptor which consists of the amino acid sequence represented by SEQ ID NO: 1 or SEQ ID NO: 2, wherein the amino acid sequencehas deletion, substitution, or addition of one or several amino acids, is distributed on the surface of an enteroendocrine cell (for example, an L cell) or an enteroendocrine cell line (for example, STC-1 orNCI-H716 cell or GLUTag cell line), and binds to a ligand therefor to convey, into a cell, a signal for GLP-1 secretion.

The GT01 polypeptide gene according to the invention may be cloned from, for example, a cDNA library or a genomic DNA library using PCR primers prepared based on the sequence information deposited in a public database (NCBI) (accession number: NM_181748 (mouse), NM_181745 (human), etc.).

The PCR primer can be designed using a primer design software program such as Primer 3 (Whitehead Institute for Biomedical Research). In addition, the PCR primer can be synthesized using a standard synthesis technique employing, for example, an automatic DNA synthesizer, but may be obtained from a commercial source. The amplified product expected to result from a PCR reaction preferably has such a length that amplification is efficient and subsequent separation by agarose gel and base sequence analysis are easy, and the design is preferably performed so that it has a length of, for example, 80 to 200 bases. The PCR reaction is carried out employing the prepared PCR primer and using a cDNA library or the like as a template, and it is confirmed e.g., by sequencing that the amplified product obtained is a desired product.

The cDNA library used here may be prepared from cells of any animal including human (for example, human, mouse, rat, guinea pig, chick, rabbit, pig, cattle, monkey, sheep, dog, or cat), including, but not limited to, immune system cells, hematocytic cells, fibroblasts, splenocytes, hepatocytes, marrow cells, pancreatic cells, Langerhans cells, epithelial cells, muscle cells, nerve cells, glia cells, fat cells, or established cell lines thereof or precursor cells thereof, or may be prepared from a tissue of any animal (for example, human, mouse, rat, guinea pig, chick, rabbit, pig, cattle, monkey, sheep, dog, or cat), including, but not limited to, brain, spinal cord, pituitary gland, thymus, peripheral blood, spleen, lymphatic tissue, pituitarygland, stomach, pancreas, kidney, genital gland, thyroid gland, gall bladder, testis, orchis, ovary, placenta, uterus, bone, joint, or skeletal muscle. The preparation of the cDNA library may be carried out using a conventional technique in the art (see, for example, Sambrook, et al., 1989).

### 2. A ligand for the GT01 polypeptide

As used herein, "ligand" refers to a chemical molecule fitting a receptor, and includes both concepts of "agonist" and "antagonist".

In addition, "agonist" as used herein includes any of the molecules inducing the biological activities of an endogenous GT01 polypeptide (the activities of binding to a ligand to convey, into a cell, a signal for GLP-1 secretion) . On the other hand, "antagonist" includes any of the molecules competing with an agonist to inhibit and neutralize part or all of the biological activities of an endogenous GT01 polypeptide. According to the invention, "agonist" may particularly have, but not limited to, the effect of promoting GLP-1 secretion.

### (1) Identification of a ligand

When a compound such as the one to be tested is added to an assay system for detecting the biological activities of a GT01 polypeptide (the activities of binding to a ligand to convey, into a cell, a signal for GLP-1 secretion), the compound is an agonist if the biological activities of the GT01 polypeptide are promoted, and conversely, the compound is an antagonist if the activities are suppressed. Specifically, by way of non-limiting example, when, in the determination of intracellular calcium concentration shown in Examples to be described, a test compound is allowed to contact with a cell used in the assay, it can be deemed that the test compound is an agonist if the intracellular calcium concentration is increased and the compound is an antagonist if the increase of the intracellular calcium concentration is inhibited even in the presence of an agonist.

### (2) Method for identifying a ligand

The method for determining a ligand according to the invention is **characterized in that** there are determined the binding amount of a candidate substance (test substance) to the GT01 protein of the invention or a partial peptide thereof, the cell-stimulating activities thereof, and the like when the GT01 protein or the partial peptide is allowed to contact with the candidate substance. More specifically, the invention provides: (i) a method for identifying a ligand for the GT01 protein or a salt thereof, characterized by comprising determining the binding amount of a labeled candidate substance to the GT01 protein or a salt thereof or a partial peptide of GT01 or a salt thereof when the labeled test compound is allowed to contact with the GT01 protein or a salt thereof or a partial peptide of GT01 or a salt thereof; (ii) a method for identifying a ligand for the GT01 protein, characterized by comprising determining the binding amount of a labeled candidate substance to a cell containing the GT01 protein or a membrane fraction of the cell when the labeled candidate substance is allowed to contact with the cell or the membrane fraction; (iii) a method for identifying a ligand for the GT01 protein or a salt thereof, characterized by comprising determining the binding amount of a labeled candidate substance to the GT01 protein or a salt thereof when the labeled candidate substance is allowed to contact with the GT01 protein expressed, by culturing a transformant containing DNA encoding the GT01 protein, on the cell membrane; (iv) a method for identifying a ligand for the GT01 protein or a salt thereof, characterized by comprising determining the activities of stimulating a cell containing the GT01 protein (for example, intracellular calcium concentration) via the GT01 protein when a candidate substance is allowed to contact with the cell; and (v) a method for identifying a ligand for the GT01 protein of the invention or a salt thereof, characterized by comprising determining cell-stimulating activities (for example, intracellular calcium release, intracellular cAMP formation, intracellular cGMP formation, inositol phosphate production, cell membrane potentialfluctuation, and intracellular protein phosphorylation) via a receptor protein when a candidate substance is allowed to contact with the receptor protein expressed, by culturing the transformant containing DNA encoding GT01, on the cell membrane.

The GT01 protein used in the method for identifying a ligand according to the invention may be any protein containing a GT01 protein or a partial peptide thereof, but is particularly preferably a GT01 protein abundantly expressed using animal cells.

### (3) Antisense RNA or DNA

An antisense RNA or DNA against a GT01 gene has the possibility of acting as an effective antagonist. The antisense RNA or DNA molecule hybridizes with a target mRNA for inhibiting translation to inhibit the function of a target factor. The antisense RNA is, for example, designed so as to hybridize with mRNA in vivo to inhibit translation from the mRNA into a GT01 polypeptide (Okano, et al., 1991). The DNA oligonucleotide is designed, for example, so as to be complementary to the transcription initiation region of the GT01 gene, resulting in the inhibition of GT01 expression (Cohen, 1989).

The antisense RNA or DNA can be introduced into a cell in such a way that they can be expressed in vivo so as to inhibit the expression of the GT01 polypeptide. When the antisense DNA is used, it is preferably, for example, an oligonucleotide binding to a position falling within the range of about -10 to +10 bases from a target gene sequence.

### (4) Anti-GT01 polypeptide antibody

The invention includes an antibody capable of specifically binding to the GT01 polypeptide, and a fragment thereof such as Fab or (Fab)₂.

As used herein, "antibody" (anti-GT01; including an agonist, antagonist, or neutralizing antibody) includes a monoepitope-specific anti-GT01 polypeptide antibody, a polyepitope-specific anti-GT01 polypeptide antibody, or a single chain antibody thereof, or a fragment thereof.

These antibodies include, for example, a monoclonal antibody, a polyclonal antibody, and a humanized antibody.

### (4)-1. Polyclonal antibody

The polyclonal antibody can be prepared, for example, by injecting a mixture of an immunogen and an adjuvant into a mammal host animal. Typically, the immunogen and/or adjuvant are subcutaneously or intraperitoneally injected plural times to the host animal. Examples of the immunogen include fusions of the GT01 polypeptide to polypeptides heterologous thereto, or fragments thereof. Examples of the adjuvant include Freund's complete adjuvant and monophosphoryl lipid A-synthetic trehalose dicorynomicolate (MPL-TDM). To enhance an immune response, the immunogen may be injected after binding to a protein having immunogenicity suchas keyhole limpet heamocyanin (KLH), serumalbumin, bovine thyroglobulin, or soybean trypsin inhibitor.

Alternatively, the preparation may be carried out using chicken producing an IgY molecule (Schade, et al., 1996).

For details regarding methods for producing antibodies, see, for example, Ausubel, et al., 1987 or Harlow and Lane, 1988.

### (4)-2. Monoclonal antibody

The anti-GT01 polypeptide monoclonal antibody is prepared using a hybridoma method (Milstein and Cuello, 1983).

This method comprises the following 4 steps: (i) immunizing a host animal or lymphocytes derived from the host animal; (ii) recovering lymphocytes secreting (orpotentially secreting) monoclonal antibodies; (iii) fusing the lymphocytes to immortalized cells; and (iv) selecting cells secreting a desired monoclonal antibody (anti-GT01 polypeptide).

A mouse, rat, guinea pig, hamster, or another suitable host animal is selected as an animal for immunization and injected with an immunogen. Alternatively, lymphocytes obtained from the animal for immunization may be immunized in vitro. When human cells are desirable, peripheral blood lymphocytes (PBLs) are generally used. However, spleen cells or lymphocytes derived from other mammal animals are more common and preferable. Examples of the immunogen include GT01 polypeptide and fusions of the GT01 polypeptide to polypeptides heterologous thereto, or fragments thereof.

The lymphocyte obtained from a host animal after immunization is fused to an immortalized cell line using a fusing agent such as polyethyleneglycol in order to establish a hybridoma cell (Goding, 1996). As a cell for fusion, the myeloma cell of a rodent, cattle, or human, immortalized by transformation or the myeloma cell line of a rat or mouse is used. After cell fusion, cells are grown in a suitable culture medium containing one or pleural substrates inhibiting the growth or survival of non-fused lymphocyte and immortalized cell line. A typical technique uses a parent cell lacking the enzyme hypoxanthine guanine phosphoribosyltransferase (HGPRT or HPRT). In this case, hypoxanthine, aminopterin, and thymidine inhibit the growth of HGPRT-deficient cells, and are added to a medium allowing the growth of hybridoma cells (HAT medium).

In preparing the monoclonal antibody, a preferable immortalized cell line is a mouse myeloma strain which is available from American Type Culture Collection (Manassas, VA). For the production of monoclonal antibodies using human myeloma and mouse-human heteromyeloma cell lines, see Kozbor, et al., 1984.

Since a hybridoma cell extracellularly secretes an antibody, whether a monoclonal antibody to the GT01 polypeptide has been produced or not can be confirmed using a liquid culture medium. The binding specificity of the monoclonal antibody produced can be evaluated by an immunoprecipitation technique such as radioimmunoassay (RIA) or enzyme linked immunosorbent assay (ELISA) or by in vitro binding assay (Harlow and Lane, 1988; Harlow and Lane, 1999).

The anti-GT01 polypeptide monoclonal antibody-secreting hybridoma cells can be isolated as a single clone by a limiting dilution method and subculture (Goding, 1996). Suitable culture media include a Dulbecco's modified Eagle medium, RPMI-1640, and, in some cases, a protein-free medium or a serum-free medium (for example, Ultra DOMA PF or HL-1; Biowhittaker; Walkersville, MD). In addition, the hybridoma cells may be proliferated in the ascites fluid of a suitable host animal.

The monoclonal antibody is isolated and purified from the medium or ascites fluid using a method well-known to those skilled in the art, such as protein A sepharose, hydroxyapatite chromatography, ammonium sulfate precipitation, or affinity chromatography (Harlow and Lane, 1988; Harlow and Lane, 1999) .

The monoclonal antibody may be also prepared by a gene recombination technique (U.S. Patent No. 4,166,452). To identify a gene encoding a desired monoclonal antibody polypeptide from a hybridoma cell line secreting an anti-GT01 polypeptide antibody, for example, oligonucleotide probes specifically binding to mouse heavy-chain and light-chain antibody genes may be used. As a result, when the heavy-chain and light-chain antibody genes are obtained, these genes can be sequenced to identify a desired antibody gene. In order to express a monoclonal antibody, the DNA fragment of the identified and isolated antibody gene is introduced into a suitable expression vector, and the vector is transfected into a host cell not producing other Ig proteins such as simian COS-7 cell, Chinese hamster ovary (CHO) cell, or myeloma cell. The isolated DNA fragment may be modified, for example, by replacing the coding sequences of the constant domains of the human heavy-chain and light-chain with homologous mouse sequences (U. S. Patent No. 4, 816, 567; Morrison, et al., 1987), or by fusing part or all of the sequence encoding a non-Ig polypeptide to the coding sequence of Ig. The non-Ig polypeptide can be replaced by the constant domain of antibody or the constant domain of the antigen-binding site to prepare a chimeric divalent antibody.

### (4)-3. Humanized and human antibodies

The GT01 polypeptide antibody includes a humanized or human antibody. The humanized form of a non-human antibody is a chimeric Ig, Ig chain, or fragment (Fv, Fab, Fab', F(ab')₂, or the antigen binding region of a different antibody) containing a minimal sequence derived from a non-human Ig.

A humanized antibody generally has one or pleural amino acid residues introduced from a non-human-derived Ig. These non-human amino acid residues are often selected from the variable domain. The humanized antibody can be prepared, for example, by replacing the CDRs or CDR sequences of a mouse with corresponding human antibody sequences (Jones, et al., 1986; Riechmann, et al., 1988; Verhoeyen, et al., 1988). In other words, the humanized antibody typically means a human antibody in which particular CDR residues in the human Ig are replaced by CDR residues derived from the corresponding site of a mouse. The humanized antibody includes a human Ig substituted by residues having a desired specific affinity to an antigen, which construct a CDR of a non-human species such as mouse, rat, or rabbit. In addition, the Fv framework residues of a human Ig may be substituted by non-human-derived residues (Jones, et al., 1986; Presta, 1992); Riechmann, et al., 1988).

### (5) A ligand for the GT01 polypeptide

Available methods for determining a ligand include, when the GT01 receptor protein is allowed to contact with a test compound (candidate ligand), for example, a method for checking for the intracellular migration of the receptor protein and a method for determining the activities of stimulating a cell expressing the receptor protein, which are operable within the scope of technological common sense to those skilled in the art.

Specifically, whether or not a test compound can function as a ligand (agonist or antagonist) can be examined, for example, by expressing, on the surface of a test cell, an expression vector for expressing a chimeric fusion protein between the GT01 receptor protein according to the invention and a fluorescent protein (e.g., GFP, CFP, YFP, or DsRED) and observing, by a fluorescence microscope or the like, the intracellular migration of the chimeric protein when the test compound is allowed to contact the expressed vector. The vector for expressing the chimeric fusion protein with the fluorescent protein can be constructed by inserting, into a vector into which a commercial fluorescent protein gene (e.g., pDsRed, pEGFP, or pCFP; Clontech) is inserted, the GT01 gene of the invention so as to be coincident with a frame.

When a test compound is allowed to contact with a cell on the surface of which the GT01 receptor protein is expressed, the cell-stimulating activities (e.g., arachidonic acid release, acetylcholine release, intracellular Ca²⁺ release, intracellular cAMP formation, inositol phosphate production) thereof via the receptor protein may be also detected to identify a candidate ligand. The method for determining the concentration of free intracellular Ca²⁺ is used in the invention. The concentration of intracellular Ca²⁺ can be determined using a technique well-known to those skilled in the art. By way of example, a method employing a fluorescent substance emitting fluorescence by binding to Ca²⁺ is generally often used.

Preferred methods of the invention for identifying a ligand are a method based on intracellular migration using chimeric fusion between a fluorescent substance and the GT01 receptor protein and a method for detecting a change in the concentration of intracellular Ca²⁺.

### 3. Cell culturing

To identify a ligand for the GT01 polypeptide of the invention, the polypeptide needs to be expressed on the surface of a suitable cell in such a state that it can function.

The cell which can be used here may be any cell that is a mammalian cell or an established cell line thereof. A skilled artisan can easily select a suitable cell or cell line. By way of example, a CHO, STC-1, GLUTTag, or HEK cell can be used.

In culturing cells, an MEM medium containing about 5 to 20% of fetal bovine serum and a DMEM medium may be, for example, used as a medium, and, if desired, glucose, glutamine, antibiotic, or the like is properly added. It is preferred that the pH is about 6 to 8; the temperature, about 37°C; and the concentration of CO₂, about 5%.

### 4. Expression of the GT01 polypeptide in mammalian cells

To identify the ligand of the invention, it is necessary that the GT01 polypeptide be expressed in suitable mammalian cells and be distributed on the surface of the cells.

To obtain a stable expression strain for the GT01 polypeptide, a DNA encoding the GT01 polypeptide of the invention (including a substantially identical polypeptide or a partial peptide thereof; hereinafter referred to the same) alone or a fusion polypeptide with another protein (e.g., GFP or G16) is inserted into a suitable vector (e.g., pCDN), which is then introduced into desired cells.

Methods for introducing the vector into animal cells include a DEAE-dextran method (Lopata, et al., 1984), an electroporation method, a calcium phosphate method (Chen and Okayama, 1988), and a method using a cationic lipid (Elroy-Stein and Moss, 1990).

Available markers for selecting a recombinant cell stably transformed include, but not limited to, a hygromycin-resistant marker (Hyg^{r}), a dihydrofolate reductase gene (dhfr), an ampicillin-resistant gene (Amp^{r}), a kanamycin-resistant gene (Kan^{r}), and a neomycin-resistant gene (Neo^{r}, G418).

### 5. A pharmaceutical composition containing an agonist or antagonist for the GT01 polypeptide.

An agonist or antagonist for the GT01 polypeptide can be used, together with a pharmacologically acceptable vehicle, as a therapeutic agent in the form of a pharmaceutical composition having no negative effect on the living body.

The term "pharmacologically acceptable vehicle" refers to a vehicle including a solvent, a dispersion medium, a coating agent, antibacterial and antifungal agents, and an agent retarding adsorption by isotonic action or a mimetic thereof, and suitable for pharmaceutical administration (Gennaro, 2000) . Examples of the vehicle and a preferable diluent for the vehicle include, but not limited to, water, saline, finger solution, dextrose solution, and 5% human serum albumin. A water-insoluble medium such liposome or fixed oil is also used. In addition, a particular compound which protects or promotes the activities of the GT01 polypeptide of the invention and an agonist or antagonist for the polypeptide, including an anti-GT01 polypeptide antibody can be incorporated into the composition.

### (1) Preparation of the pharmaceutical composition

The pharmaceutical composition of the invention is formulated so as tobe compatible with therapeutically suitable administration routes including intravenous or oral administration and direct administration into the stomach. The solution or suspension used for intravenous administration or direct administration into the stomach may contain, but not limited to, a sterile diluent such as water for injection, saline, fixed oil, polyethylene glycol, glycerin, propylene glycol, or other synthetic solvent; a preservative such as benzyl alcohol or other methylparabens; an antioxidant such as ascorbic acid or sodium bisulfite; a soothing agent such as benzalkonium chloride or procaine hydrochloride; a chelating agent such as ethylenediaminetetraacetic acid (EDTA); a buffer such as acetate, citrate, or phosphate; and an agent for adjusting osmotic pressure such as sodium chloride or dextrose.

The pH may be adjusted using an acid or base such as hydrochloric acid or sodium hydroxide. A parenteral preparation is contained in an ample, a glass or plastic disposable syringe, or a vial for multiple administrations.

### (2) Injectable preparations

Pharmaceutical compositions suitable for injection include a sterilized injectable solution or dispersion medium, wherein it is a sterile aqueous solution (water-soluble) or dispersion medium for preparation at the time of use, and a sterilized powder (including a lyophilized protein or nucleic acid). Suitable vehicles for intravenous administration include saline, bacteriostatic water, Cremophor EL^{™} (BASF, Parsippany, N.J.), and phosphate buffered saline (PBS). When used as an injection, the composition must be sterile, and must retain sufficient fluidity because it is administered using a syringe. The composition must be stable e.g., to chemical change and corrosion, and must not produce contamination from microorganisms such as bacteria and fungi during preparation and storage. The vehicle used here may be a solvent, including, for example, water, ethanol, polyol (e.g., glycerol, propylene glycol, or liquid polyethylene glycol), or a suitable mixture, or a dispersion medium. By way of example, a coating agent such as lectin is used to maintain a particle size as required for the dispersion medium, and a surfactant is used to keep moderate the fluidity. Various antibacterial and antifungal agents such as, for example, paraben, chlorobutanol, phenol, ascorbic acid, and thimerosal can be used to prevent the contamination of microorganisms. An agent keeping isotonicity such as polyalcohol (e.g., sugar, mannitol, or sorbitol) or sodium chloride may be contained in the composition. The composition capable of retarding adsorption contains an agent such as aluminum monostearate or gelatin.

The sterile injectable solution is prepared by adding necessary components alone or a combination thereof with other components to a suitable solvent, to which a necessary amount of an active compound is then added before sterilization. The dispersion medium is typically prepared by incorporating an active compound into a sterile medium containing a basic dispersion medium and the above-described other necessary components. Methods for preparing a sterile powder for preparing a sterile injectable solution include vacuum drying and freeze drying for preparing a powder containing an active ingredient and any desired component derived from the sterile solution.

### (3) Oral compositions

The oral composition typically contains an inactive diluent, or a vehicle which is harmless even when incorporated into the body. The oral composition is, for example, included in a capsule of gelatin, or pressurized for tabletation. For oral treatment, an active compound is incorporated together with an excipient, and used in the form of a tablet, troche, or capsule. The oral composition can be also formulated using a flowable vehicle, and the composition in the flowable vehicle is orally applied. In addition, a pharmaceutically suitable binder and/or an adjuvant substance or the like maybe included.

The tablet, pill, capsule, troche, and the like can contain the following components, or compounds having similar properties: an excipient such as microcrystalline cellulose; a binder such as gum arabic, tragacanth, or gelatin; a swelling agent such as starch, lactose, alginic acid, Primogel, or corn starch; a lubricant such as magnesium stearate or Strrotes; a lubricating agent such as colloidal silicon dioxide; a sweetening agent such as sucrose or saccharin; and a flavoring agent such as peppermint, methylsalicylic acid, or orange flavor.

### (5) Systemic administration

The systemic administration may be carried out transmucosally or percutaneously. For the transmucosal or percutaneous administration, a penetrant capable of permeating a target barrier is selected. Transmucosal penetrants include surfactants, bile salts, and fusidic acid derivatives. A transnasal spray or a suppository may be used for the transmucosal administration. For the transmucosal administration, an active compound is formulated in the form of an ointment, salve, gel, or cream.

For delivery to the rectum, a compound may be also formulated in the form of a suppository (together with, for example, cocoa butter and another base such as glyceride) or a retentive enema.

### (6) Vehicles

The GT01 polypeptide of the invention and an agonist or antagonist for the polypeptide, including an anti-GT01 polypeptide antibody may be formulated in the form of a controlled release preparation such as an implant or a microencapsulated delivery system, using a vehicle capable of preventing the immediate removal thereof from the body. Biodegradable, biocompatibile polymers such as ethylene vinyl acetate, polyethylene glycol, polyacid anhydride, polyglycolic acid, collagen, polyorthoester, and polylactic acid may be used. These materials can be obtained from ALZA Corporation (Mountain View, CA) or NOVA Phamaceuticals, Inc. (Lake Elsinore, CA), and also can be readily prepared by a skilled artisan. A suspension of liposome may be also used as a pharmaceutically acceptable vehicle. By way of non-limiting example, a useful liposome is prepared as a lipid composition containing phosphatidylcholine, cholesterol, and PEGylated phosphatidylethanol (PEG-PE) by passing through a filter having a suitable pore size so as to provide a size suited for use, and purified using a reverse-phase evaporation method. By way of example, the Fab' fragment of antibody or the like may be also bound to the liposome via disulfide exchange reaction (Martin and Papahadjopoulos, 1982). For details regarding methods for the preparation, see, for example, descriptions in Eppstein, et al., 1985; Hwang, et al., 1980.

### (7) Dosages

In the treatment or prevention of a particular disease using the GT01 polypeptide of the invention, a gene encoding the polypeptide, or the like, suitable dosage levels thereof depend on the condition of a patient to be treated, the method of administration, and the like, but can be easily optimized by one of skill in the art.

For administration by injection, for example, a daily dose of about 0.1 µg to 500 mg per kg of body weight of a patient is preferably administered, and may be generally given at a time or in divided portions. The dosage level is preferably about 0.1 µg/kg to about 250 mg/kg per day, more preferably about 0.5 to about 100 mg/kg.

For oral administration, the composition is preferably provided in the form of a tablet containing 1.0 to 1,000 mg of active ingredient, and the amount of the effective active ingredient present in a dose for a patient to be treated is preferably 1.0, 5.0, 10.0, 15.0, 20.0, 25.0, 50.0, 75.0, 100.0, 150.0, 200.0, 250.0, 300.0, 400.0, 500.0, 600.0, 750.0, 800.0, 900.0, or 1,000.0 mg. The compound is administered with a dosage regimen of once to four times a day, preferably once to twice a day.

### (8) Unit dosage

The pharmaceutical composition or preparation must comprise a uniform unit dosage to secure a constant dosage. The unit dosage means a unit containing a single dose effective for treating a patient and formulated together with a pharmaceutically acceptable vehicle. The determination of the unit dosage of the invention is influenced, for example, by the physical and chemical characteristics of a compound (e.g., free fatty acid or anti-GT01 polypeptide antibody) to be formulated, the expected therapeutic effect thereof, and points of concern in formulation typical for the compound.

### 6. Gene therapy composition

To introduce the nucleic acid molecule (for example, a vector into which a polynucleotide encoding the GT01 polypeptide is inserted) disclosed in the invention into cells of a patient, there are two major methods, in vivo and ex vivo. For in vivo delivery, it is directly injected into the site of a patient required to be treated. For ex vivo treatment, cells at the site of a patient required to be treated can be isolated, into which a formulated nucleic acid molecule is then introduced, followed by administering the resultant cells directly or, for example, after encapsulation in a porous membrane to be embedded in the patient (see U.S. Patent Nos. 4,892,538 and 5,283,187). A technique available for introducing the nucleic acid molecule into live cells is selected depending on whether it is introduced into cultured cells or the like in vitro or into a patient invivo. Techniques suitable for introducing the nucleic acid molecule into mammalian cells in vitro include liposome, electroporation, microinjection, transfection, cell fusion, DEAE-dextran method, and calcium phosphate method. The transfection comprises the binding of the particle of a recombinant virus (preferably, retrovirus) to a cellular receptor and the subsequent introductionof the nucleic acidmolecule contained in the particle into the cell. The vector typically used for the ex vivo delivery of a gene is retrovirus.

Current preferred techniques for in vivo nucleic acid transfer include systems using a viral or non-viral vector (adenovirus, lentivirus, Herpes simplex I virus, or adeno-associated virus (AAV)) and a cationic lipid-based system (a lipid useful for the lipid-mediated transfer of a gene is, forexample, DOTMA, DOPE, orDC-Chol; see, forexample, Tonkinson, et al., Cancer Investigation, 14 (1) : 54-65 (1996)). The most preferable vectors for use in gene therapy are viruses; among others, adenovirus, AAV, lentivirus, or retrovirus is most preferable. Viral vectors such as a retroviral vector contain at least one transcriptional promoter/enhancer or positioning factor. In addition, for example, in the case of transcription in a state containing a gene encoding the G01 polypeptide, viral vectors such as a retroviral vector contain a cis element enabling the translation of the coding gene, that is, a nucleic acid sequence functioning as a translation initiation sequence. These vector constructs contain a packaging signal or long terminal repeat (LTR) or portion thereof suitable for the virus used. In some cases, the vector constructs also contain polyadenylation and translational termination sequences. For example, they contain a 5'LTR, tRNA-binding site, a packaging signal, a DNA synthesis initiation point, and a 3' LTR or portion thereof. Other non-viral vectors may be, for example, cationic lipids, polylysines, or dendrimers.

In some cases, the vector is preferably provided together with an agent targeting a nucleic acid used for treatment at desired cells, such as, for example, an antibody specific for a cell-surface membrane protein. For review of the currently known gene labeling and gene therapy protocols, see Anderson, et al., Science, 256: 808-813 (1992). For suitable gene therapy and methods for making retroviral particles and structural proteins, see U.S. Patent No. 5,681,746.

### 7. A kit for the pharmaceutical composition

The pharmaceutical composition may be included together with an explanation for administration in a kit, container, or pack. When the pharmaceutical composition according to the invention is supplied in the form of a kit, the different constituents of the pharmaceutical composition are packaged in separate containers, and mixed immediately before use. The separate packaging of constituents is intended to enable the long-term storage thereof without the loss of function of an active constituent.

### (1) Containers

Reagents contained in the kit are provided in such a kinds of container that the constituents effectively maintain the activities thereof for a long period of time, are not adsorbed to the material of the container, and cause no deterioration. By way of example, a sealed glass ample contains a buffer packaged under a neutral and unresponsive gas such as nitrogen. An ample is composed of glass, an organic polymer such as polycarbonate or polystyrene, ceramic, metal, or any of other suitable materials typically used for holding reagents. Other suitable containers include, for example, a simple bottle made of a material similar to that of an ample or the like, and a packaging material whose inside is lined with a foil such as aluminium or alloy. Other containers include a test tube, a vial, a flask, a bottle, a syringe, and a container similar thereto. Containers have a sterile access port, such as a bottle having a stopper which can be pierced by a hypodermic injection needle.

### (2) Instructions

The kit also includes instructions. Instructions for the use of the kit comprising the pharmaceutical composition may be printed on paper or other materials, or supplied in the form of an electrically or electromagnetically readable medium such as floppy™ disk, CD-ROM, DVD-ROM, Zip disk, video tape, or audio tape. Detailed instructions may be actually included in the kit, or may be carried on a website designated or informed through an electronic mail system or the like by the manufacturer or distributor of the kit.

### 8. A dietary supplement composition or dietary supplement food containing an agonist or antagonist for the GT01 polypeptide.

When an agonist or antagonist for the GT01 polypeptide is used to make the dietary supplement composition or dietary supplement food according to the invention, generally, the shape of the food is not particularly restricted, but is preferably that enabling the long-term ingestion thereof as an ordinary food; examples thereof can include a tablet, a granule, a powder, a refreshing drink, confectionary, bread, and margarine. In addition, an additive, extender, perfume, sweetener, thickener or the like usually used in foods may be properly mixed as far as advantages of the invention are not impaired.

### 9. Other medical and pharmaceutical applications of the GT01 protein or a partial peptide thereof

The GT01 polypeptide of the invention or a partial peptide thereof, or a salt thereof and polynucleotides encoding them can be used, for example, for: (i) the determination of a ligand (agonist) for the GT01 protein; (ii) an agent for preventing and/or treating diseases associated with the dysfunction of the GT01 protein; (iii) an agent for gene diagnosis; (iv) the quantitative determination of the ligand for the GT01 protein; (v) the screening of a compound altering the binding properties of the ligand to the GT01 protein; (vi) an agent for preventing and/or treating various diseases, containing the compound altering the binding properties of the ligand to the GT01 protein; (vii) the quantitative determination of the GT01 protein or a partial peptide thereof; (viii) the neutralization of the GT01 protein or a partial peptide thereof by an antibody thereto; and (ix) the preparation of a non-human animal having a gene encoding the GT01 protein. Particularly, a receptor binding assay system employing an expression system for the recombinant GT01 protein of the invention may be used to screen a substance (for example, agonist or antagonist) altering the binding properties of a human- or mammal-specific GT01 protein to a ligand therefor; the resultant agonist or antagonist can be used, for example, as an agent for preventing or treating various diseases.

Specifically, the GT01 protein of the invention or a partial peptide of the same, or a salt thereof is useful as a reagent for screening or identifying a ligand (agonist) or antagonist for the GT01 protein. These reagents can each provide a component of a kit for screening a substance altering the binding properties of the ligand to the GT01 protein. As described above, the present invention provides a method for determining a ligand (agonist) or antagonist for the GT01 protein, **characterized in that** the GT01 protein or a partial peptide of the same, or a salt thereof is allowed to contact with a test substance. Examples of the test substance include human or mammal (e.g., mouse, rat, pig, cattle, sheep, or monkey) tissue extracts, cell culture supernatants, and artificially synthesized compounds.

To perform a method for determining a ligand for the GT01 protein of the invention or a salt thereof, an appropriate GT01 protein fraction and a labeled test substance are used. Preferably, the GT01 protein fraction is, for example, a natural GT01 protein fraction or a recombinant GT01 protein fraction having an activity comparable to that thereof. To determine a ligand for the GT01 protein or a salt thereof, for example, cells or membrane fractions thereof containing the GT01 protein are first suspended in a buffer suitable for the determination method to prepare a GT01 preparation. The buffer may be any buffer which does not inhibit the binding of a ligand to a receptor protein, including a phosphate buffer or a Tris-hydrochloric acid buffer. For the purpose of reducing non-specific binding, a surfactant such as CHAPS, Tween-80, or deoxycholate, or a protein such as bovine serum albumin or gelatin may be also added to the buffer. A given amount of radiolabeled test substance is allowed to coexist in a solution containing the GT01 protein. For the purpose of knowing the amount of non-specific binding, a reaction tube to which a large excess of the unlabeled test compound has been added is also provided. The reaction is conducted at about 4 to 50°C, preferably about 4 to 37°C for about 10 minutes to 24 hours, preferably about 30 minutes to 3 hours. After the reaction, filtration and subsequent washing with an appropriate amount of the same buffer are carried out, followed by measuring the remaining radioactivity on the filter paper using a liquid scintillation counter or the like. A test substance for which the count corresponding to the total amount of binding, less the amount of non-specific binding exceeds 0 cpm can be selected as a ligand (agonist) for the GT01 protein of the invention or a salt thereof.

The following Examples are provided for illustrative purposes only, and not intended to limit the scope of the invention in any manner.

All patent documents and references cited in this specification are incorporated herein by reference in their entirety.

### Example 1

### Cloning of a GT01 gene

cDNA was prepared by subjecting 5 µg of total ileal RNA in a human organ RNA panel or total RNA extracted from mouse ileum to reverse transcription using Random primer (from Takara) according to a method included with SuperScipt II (from Invitrogen). After the reaction, a 5' primer (5'-ATGTCCCCTGAATGCGCGCGGG-3') (SEQ ID NO: 3) and a 3' primer (5'-GCCAGAAATAATCGACAAGTCA-3') (SEQ ID NO: 4) were employed to perform RT-PCR using TaKaRa EX Taq (from Takara). PCR reaction was carried out by denaturing the cDNA at 95°C for 2 minutes and then conducting 35 reaction cycles of 96°C for 30 seconds, 52°C for 30 seconds, and 72°C for 2 minutes to amplify the PCR product. Then, the resultant product was subjected to elongation reaction at 72°C for 5 minutes, and the reaction was terminated by cooling to 4°C. The PCR fragment was subcloned into pGEM-T easy (from Promega) vector and then sequenced. Each of the human and mouse fragments was cut out with a restriction enzyme, and the whole thereof was placed downstream of the promoter of an expression vector, pIRES (from Clonetech) so as to express the full length. Further, in conducting PCR, primers from which stop codons were removed were prepared, and a full-length cDNA was produced in the same procedure. The resultant cDNA was introduced into a pEGFP-N3 expression (from Clonetech) vector or the expression vector whose EGFP sequence was replaced by G16 to make an expression vector capable of producing a fusion protein with EGFP or G16.

### Example 2

### Tissue distribution of gene expression

### (1) Preparation of tissues

C57BL/6 male mice were anaesthetized with ether, and subjected to perfusion fixation using 4% paraformaldehyde/0.1M phosphate buffer (pH 7.4). Then, part of the colon was taken, the content thereof was removed in cooled phosphate-buffered saline (PBS), and fixation was then carried out at 4°C for one day. Subsequently, replacement was performed with 20% sucrose/0.1M phosphate buffer (pH 7.4) at 4°C for two days or more. The sample subjected to the replacement was treated with O.C.T Compound and frozen using liquid nitrogen and stored at -80°C until use. A fresh frozen sample was prepared as described below. Male mice of the same strain were anaesthetized with ether, part of the jejunum or colon was taken, and the intestinal content was then washed with cooled PBS. The water was slightly drained from the sample, which was then rapidly embedded using O.C.T Compound, followed by freezing with liquid nitrogen and storing at -80°C until use.

### (2) RT-PCR

Total RNA was extracted from each eviscerated mouse organ using ISOGEN (Nippon Gene). The resultant total RNA (5 mg) was subjected to RT reaction using Ready-To-Go You Prime First-Strand Beads (from Amersham Bioscience, Sweden) to prepare cDNA. After the reaction, a 5' primer (5'-CGCACCCGCTTTCCCTTCTTCTC-3') (SEQ ID NO: 3) and a 3' primer (5'-AGCTCT TTCCTTGATGCCTTTGTGA-3') (SEQ ID NO: 4) were employed to perform RT-PCR using TaKaRa EX Taq (from Takara) . PCR reaction was carried out by denaturing cDNA at 95°C for 2 minutes and then conducting 35 reaction cycles of 96°C for 30 seconds, 52.3°C for 30 seconds, and 72°C for 2 minutes to amplify the PCR product. Then, the resultant product was subjected to elongation reaction at 72°C for 5 minutes, and the reaction was terminated by cooling to 4°C.

### (3) Southern hybridization

After the RT-PCR reaction, the products were electrophoresed using 2% agarose gel, and transcribed to a nitrocellulose membrane. To prepare a probe for southern hybridization, the sequence of a mouse GT01 gene (SEQ ID NO: 5) cloned in pGEM-T Easy Vector (from Takara) was first cleaved with each of the restriction enzymes BssHI and BglII, and subjected to electrophoresis using 1% agarose gel. After cutting out a desired DNA band, the DNA was purified using Geneclean II (from Q-BIO gen, USA) to make a template for the probe. A mouse GT01 gene-specific DNA probe was prepared by Random Primer DNA labeling Kit Ver. 2 (from Takara) using ³²P-labeled dCTP (from NEN, USA). The ³²P-labeled DNA probe was added to the Southern blotted membrane in a hybridization buffer (5×SSC, 5×Denhart's solution, 0.5% SDS), followed by reaction overnight at 55°C. After the hybridization, the probe was washed, at 55°C for 10 minutes, with 2×SSC/0.1% SDS and then 0.2×SSC. The membrane was exposed to Fuji Imaging Plate (from Fuji Photo Film), and scanned using an image analyzer (Storm 860, Amersham Bioscience, Sweden).

### (4) Results

The results of the tissue distribution of GT01 gene expression are shown in Figure 1. The expression was observed frequently in the caecum and large intestine, relatively often in the brain and lung, and also in the rectum, pancreas, and islet cells. In addition, it was demonstrated that the gene was often expressed also in the enteroendocrine cell line STC-1. In contrast, the expression in the heart, liver, and kidney was less often observed (Figure 2).

### Example 3

### CCK immunohistochemistry (see Japanese Patent Application No. 2003-180375 specification)

A fresh frozen section of mouse jejunum was sliced into a thickness of 8 mm using a cryostat (LEICA CM1800; Leica), attached to an APS-coated slide glass (Matsunami Glass), and air-dried at -20°C. Then, the slice was fixed in Zamboni solution for 30 minutes before washing with flowing water for 10 minutes. To block endogenous peroxidase, treatment with 0.5% sodium metaperiodate was carried out for 10 minutes before washing with flowing water for 10 minutes. The non-specific reaction of an anti-CCK antibody was blocked using an antibody dilution solution (1% normal bovine serum, 0.4% Triton-X 100, PBS dilution) for one hour, followed by washing with PBS. The slide glass was transferred to a wet box, and allowed to react with a rabbit anti-CCK antibody (1:4,000, AB1972, Chemicon, USA) overnight at room temperature. After the reaction, it was subjected thrice to a 5 minutes of washing with PBS, allowed to react with a biotin-labeled goat anti-rabbit IgG (1:2,000, Cat. No. 55701, ICN Pharmaceuticals, USA) at room temperature for 2 hours, and then subj ected thrice to a 5 minutes of washing with PBS. Subsequently, an avidin-biotin-peroxidase complex (Vectastain ABC Kit, Vector Labs, USA) was allowed to react therewith for 40 minutes, followed by subjecting thrice to a 5 minutes of washing with PBS. Then, it was color-developed with a DAB reaction solution (a 50 mM Tris buffer (pH 7.6) containing 0.02% 3 , 3 -diaminobenzidine tetrahydrochloride and 0.06% hydrogen peroxide solution). After the color development, it was washed with flowing water and subjected to dehydration and penetration using an ethanol-xylene series, and the sample was then mounted using a mounting agent (MP500, Matsunami Glass).

### Example 4

### In situ hybridization

### (1) Preparation of a cRNA probe

The mouse GT01 sequence cloned in pGEM-T Easy Vector (Takara) was cleaved with a restriction enzyme, SpeI, for preparing a sense probe or with a restriction enzyme, NcoI, for preparing an anti-sense probe. The resultant linear plasmid DNAs were each used in an amount of 1 mg for cRNA probe synthesis; using DIG RNA Labeling Kit (Roche Diagnostics, Switzerland), the total amount of thereactionmixture (plasmid DNA, 1×DIG RNA labeling Mix, 1×Transcription buffer, 1 U/ml RNasin, 2U/ml T7 or SP6RNA polymerase, RNase-free dH₂O) was set to 20 ml. This reaction solution was allowed to react at 37°C for 2 hours, followed by decomposing the plasmid DNA using a DNAase before terminating the reaction employing 1 ml of 0.5M EDTA. The synthesized cRNA probe was ethanol precipitated, and the pellet obtained by centrifugation (15,000 rpm, 4°C, 15 minutes) was dried, which was then dissolved in an alkaline hydrolyzing liquid (40 mM NaHCO₃, 60 mM Na₂CO₃, pH 10.2) before subjecting to fragmentation treatment at 60°C for 9 minutes. After the treatment, ethanol precipitation was again carried out, and the precipitate was dissolved in DEPC water (Milli-Q water treated with 0.1% DEPC overnight and then heated in an autoclave at 121°C for 40 minutes for detoxication).

### (2) In situ hybridization

The frozen sample of mouse colon fixed with 4% paraform aldehyde was sectioned into a thickness of 20 mmusing a cryostat (Leica CM1800; Leica) and floated on 4×SSC (0.6 M NaCl, 0.6 M sodium citrate). The resultant section was washed with PBS and treated with 1 mg/ml Proteinase K (dilution with 0.1 M Tris-HCl (pH 8.0) /50 mM EDTA) at 37°C for 20 minutes. It was fixed with 4% paraform aldehyde for 10 minutes and then washed with PBS. This was allowed to stand in acetic anhydride (dilution with 0.1 M triethanolamine) at room temperature for 10 minutes, and again washed with PBS. Then, the probe was added to a hybridization buffer (50% formamide, 10 mM Tris-HCl (pH 7.6), 1×Denhardt Solution, 0.2 mg/ml yeast tRNA, 10% dextran sulfate, 600 mM NaCl, 0.25% SDS, 0.5 M EDTA (pH 8.0)) so as to provide a concentration of 200 ng/ml, which was allowed to react at 60°C overnight (for about 16 hours). After the hybridization reaction, the probe was washed with 2×SSC/50% formamide at 60°C for 30 minutes, which was then replaced by TNE (10 mM Tris-HCl (pH 7.6), 500 mM NaCl, 1 mM EDTA) for 10 minutes, and excess probes were digested using a 20 mg/ml RNase (dilution with TNE). Washing was carried out with TNE for 10 minutes, followed by washing with 2xSSC, 1×SSC and 0.5×SSC at 55°C for 20 minutes. To detect a signal, replacement by TBS (100 mM Tris-HCl (pH 7.5), 150 mM NaCl) was performed for 5 minutes, followed by the reaction of blocking a DIG antibody at 37°C for one hour using a 1.5% blocking reagant (dilution with TBS). Washing was carried out using TBS for 5 minutes, and antibody reaction was performed at room temperature for one hour using a goat ant-DIG antibody (Roche Diagnostics, Switzerland) 1:500 (dilution with 1.5% blocking reagent)). The antibody was removed by washing with TBST (100 mM Tris-HCl (pH 7.5), 150 mM NaCl, 0.1% Tween 20), and replacement by NTM (100 mM Tris-HCl (pH 9.5), 100 mMNaCl, 50 mM MgCl₂) was carried out for 3 minutes. Then, color development was performed under microscopic examination using 0.34 mg/ml NBT and 0.18% BCIP (dilution with NTM), and the color development reaction was terminated by treatment with a reaction termination solution (10 mM Tris-HCl, 1 mM EDTA (pH 8.0)) for 10 minutes. After the color development, the section was placed on a slide glass in PBS, mounted with 90% glycerol (dilution with PBS), and subjected to microscopic examination using an optical microscope.

### Example 5

### Preparation of stable expression cells

To obtain a vector containing a desired gene, EGFP in pEGFP-N3 (from Invitrogen) was cut out using restriction enzymes, KpnI and NotI (from Takara), and the DNA sequence of G16 was inserted employing TaKaRa Ligation Kit ver. 2 (from Takara) . In addition, the sequence of mouse GT01 was inserted upstream of G16 using the restriction enzyme, KpnI (from Takara) and TaKaRa Ligation Kit ver. 2 (from Takara).

The transfection of the DNA into cells was carried out using an electroporation method. Cells (HEK-293 (derived from human fetal kidney, 2, 500, 000 cells) were suspended in a medium (Dulbecco's Modified Eagle Medium, high glucose, GIBCO), and the DNA solution (DNA amount: 10 to 15 µg) was then added. After 10 minutes, DNAs were transfected into cells under the conditions of 240 V and 975-µF using Bio Rad Capacitance Pulse Controller Gene Pulser.

The cells transfected with the receptor DNA were cultured in an agent-containing medium (G418: 1.0 mg/mL, penicillin: 100 units /mL, streptomycin: 100 µg/mL, 10% FCS) at 37°C and 5% CO₂ for selection. After 10 days, colonies were picked up and cultured in an agent-containing medium (G418 : 0.5 mg/mL, penicillin: 100 units /mL, streptomycin: 100 µg/mL, 10% FCS).

### Example 6

### Measurement using the intracellular migration of the receptor (GT01 polypeptide)

### (1) E-C-L coating of a plate for assay

Sterile PBS (137 mM NaCl, 8.1 mM Na₂HPO₄·12H₂O, 2.68 mM KCl, 1.47 mM KH₂PO₄) containing 5 µg of E-C-L Cell Attachment Matrix (from Upstate) was added to a View Plate-96 (from Packard) so as to provide an amount of 100 µL/ well, and cultured at 37°C for one hour or at 4°C overnight. This was used for the following assay.

### (2) Seeding cells on the plate

Cells (HEK cells) stably expressing a chimeric receptor were detached with trypsin and suspended in a medium containing 10% FCS. The cells were seeded on the E-C-L- coated plate so as to provide a liquid amount of 100 µL/well and a cell number of 5×10⁴/well, and cultured overnight under conditions of 37°C and 5% CO₂, followed by removing the medium and adding a serum-free medium in an amount of 100µL/ well.

### (3) Assay of an agonist (or antagonist)

A lipid assumed to be an agonist (or antagonist) for the chimeric receptor expressed on the cell was added so as to provide an amount of 1 µL/well. Cells were maintained under conditions of 37°C and 5% CO₂ for one hour.

### Fixation and staining of cells

After culturing, the medium was removed, and a fixing and staining solution (containing 10 µg/mL Hoechst No. 33342 (from Sigma) and 2% paraformaldehyde (from Nakarai)) was added in an amount of 100 µL/well, followed by allowing to stand for 30 minutes in the dark.

Elisa Tape (from Iwaki) was affixed to the plate so as to completely cover the wells.

### (4) Assay

The ArrayScan System from Cellomics, Ltd. was used for analysis. The nucleus was stained with Hoechst and the behavior of the receptor associated with agent treatment was tracked as the behavior of a GFP-receptor chimera protein. It is known that some G-protein coupled receptors localized on cell membrane are internalized into cytoplasm by ligand stimulation. The chimeric receptors located at fixed distances from the nucleus were determined as having been internalized, and the proportion of cells in which the receptor was internalized to the total number of cells was calculated foreachwell. Based on the calculated value, it was determined whether the lipid used was an agonist (or antagonist) for the mouse GT01 receptor (Figure 3).

### Example 7

### Measurement of intracellular Ca²⁺ concentration

### (1) Measurement using FLIPR

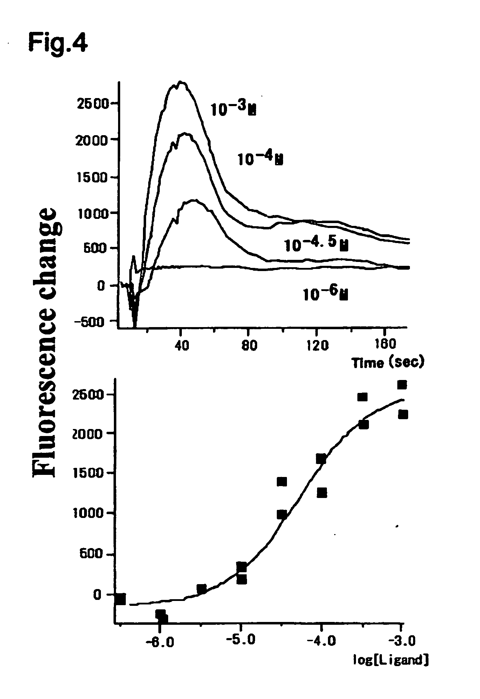

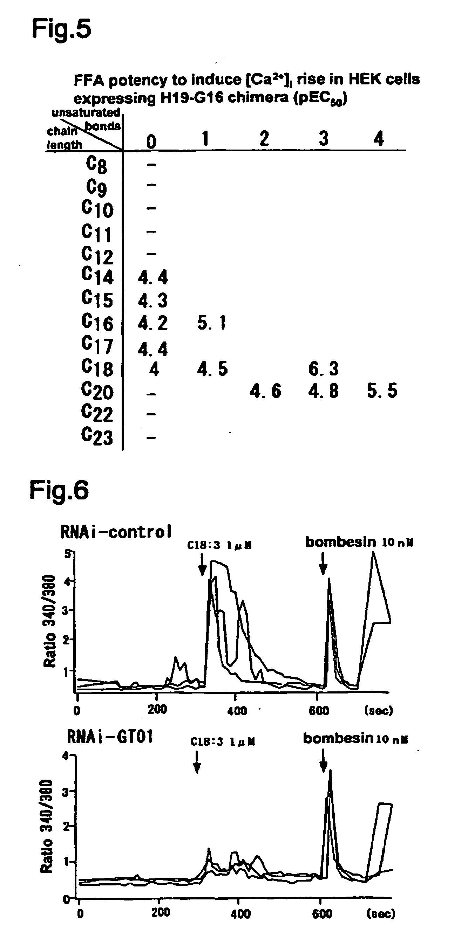

The concentration of intracellular calcium was measured as described below. Cells (HEK cells; 200,000 cells per well) on which a desired receptor was stably expressed were cultured on a 96-well plate (Collagen·Cell ware 96-well Black/Clear Plate, Becton Dickinson) for 20 hours in conditions of 37°C and 5% CO₂. FLIPR Calcium Assay Kit (from Molecular Devices) diluted with a buffer (HEPES/Hanks, pH 7.4) was added, followed by incubating at 37°C and 5% CO₂ for one hour. Diluted with the same buffer, a test agent (a free fatty acid such as capric acid, lauric acid, myristic acid, pentadecanoic acid, octanoic acid, palmitic acid, stearic acid, arachic acid, behenic acid, margaric acid, palmitoleic acid, eicosatrienoic acid, elaidic acid, petroselinic acid, oleic acid, α-linolenic acid, γ-linolenic acid, homo-γ-linolenic acid, arachidonic acid, eicosadienoic acid, eicosatrienoic acid, eicosapentaenoic acid, docosahexaenoic acid, linoleic acid, eicosatetraenoic acid, or vaccenic acid) was added, followed by measuring fluorescence intensity at 510 to 570 nm for excitation light of 488 nm using FLIPR (Fluorometric Imaging Plate Reader, Molecular Devices) (Figure 4). Figure 5 shows pEC₅₀s for the elevation of Ca²⁺ concentration in an HEK cell when various free fatty acids were added. The fatty acids shown in the data of Figure 5 are myristic acid (C14:0), pentadecanoic acid (C15:0), palmitic acid (C16:0), palmitoleic acid (C16:1), margaric acid (C17:0), stearic acid (C18:0), oleic acid (C18:1), α-linolenic acid (C18:3), eicosadienoic acid (C20:2), eicosatrienoic acid (C20:3), and eicosatetraenoic acid (C20:4). Eicosapentaenoic acid, docosahexaenoic acid, or other free fatty acids also had comparable pEC₅₀.

### (2) Measurement using CAF

Cells (2,500,000 cells) on which a desired receptor was stably expressed were suspended in 5 mL of buf fer (135 mM NaCl, 5 mM KCl, 10 mM glucose, 10 mM HEPES, 1.2 mM CaCl₂, 1 mM MgCl₂), to which 15 µL of fura2-AM was then added, followed by penetration culture at 37°C for 40 minutes. Then, a test agent was added thereto, followed by measuring the ratio of fluorescence intensities at 500 nm for two excitation waves of 340 nm and 380 nm using CAF-110 (Jasco).

### (2)-1. Microscopic measurement

Cells (STC-1 cells) are cultured in a 35-mm culture dish with a cover glass in the bottom. After washing with a Ca-tyrode solution, a Ca-tyrode solution containing 2 µM fura-AM is added thereto, which is then placed at room temperature for 20 minutes. After washing twice with a Ca-tyrode solution, 1 mL of Ca-tyrode solution is added thereto, and images are captured at room temperature at 15-second intervals using ARGUS200 (340/380 nm measurement) with a 40×objective lens. For each of the captured images, the ratio is calculated. Stimulation (using bombesin) serving as a control for the ligand is carried out 10 and 20 minutes later.

When an RNAi vector was transfected, fluorescence due to GFP simultaneously transfected is measured; a Ca²⁺ response over time for cells having GFP fluorescence and showing Ca²⁺ elevation with the control vector was quantitatively determined, and is indicated (Figure 6). The fatty acid used here is α-linolenic acid (C18:3).

### (2)-2. Preparation and transfection of an RNAi vector A pSilencer 2.1-U6 system from Ambion was used.

According to a instruction included therewith, the oligo pair selected from a desired gene was synthesized, annealed, and then ligated into the above-described vector. The resultant construct was sequenced for confirmation.

The gene transfection into STC-1 cells was carried out using Lipofectamine Plus, and intracellular Ca²⁺ concentration was then measured under a microscope (lower graph in Figure 6). As a result, the RNAi specific for GT01 eliminated the peak (upper graph in Figure 6) of elevated calcium concentration observed by the addition of α-linolenic acid (lower graph in Figure 6). Thus, it has been demonstrated that α-linolenic acid elevates intracellular calcium concentration via GT01.

### Example 8

### Measurement of CCK (See Japanese Patent Application No. 2003-180375 specification)

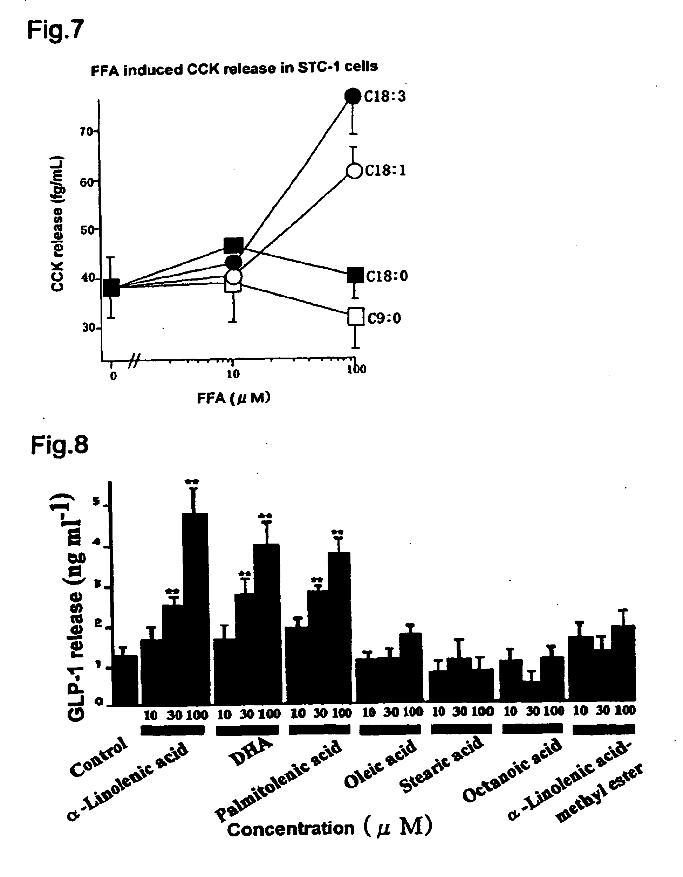

ST-1 cells were cultured in a 24-well plate at densities of 8×10⁴ and 1×10⁵ cells/cm². Cholecystokinin octapeptide (26-33, Asp-Tyr-Met-Gly-Trp-Met-Asp-Phe-NH2) was quantitatively determined 24 to 48 hours later. The cells were washed thrice with Hank's buffer (HBBS), and then reacted with various concentrations of each free fatty acid in 0.5 mL of Hank's buffer at 37°C for 60 minutes. The culture supernatant was recovered, centrifuged (at about 5, 000 g) for 5 minutes to remove cell fragments, and subjected to the measurement of CCK with a kit using the EIA method specific for CCK (26-33) (Phoenix Pharmaceuticas Inc., Belmont, CA) (Figure 7). Here, linolenic acid (C18:3), oleic acid (C18:1), stearic acid (C18:0), and pelargonic acid (C9:0) were used.

### Example 9

### Measurement of GLP-1

STC-1 cells were cultured in a 24-well plate at densities of 8×10⁴ and 1×10⁵ cells/cm². GLP-1 was quantitatively determined 24 to 48 hours later. The cells were washed thrice with Hank's buffer (HBBS), and then reacted with various concentrations (10, 30, and 100 µM) of each free fatty acid in 0.5 mL of Hank's buffer at 37°C for 60 minutes. The culture supernatant was recovered, centrifuged (at about 5, 000 g) for 5 minutes to remove cell fragments, and subjected to the measurement of GLP-1 with a kit using the EIA method specific for GLP-1 (Phoenix Pharmaceuticas Inc., Belmont, CA) (Figure 8). Here, α-linolenic acid, docosahexaenoic acid, palmitoleic acid, oleic acid, stearic acid, octanoic acid, and α-linolenic acid-methyl ester were used (Figure 8).

### Example 10

### In situ hybridization

### (1) Preparation of cRNA probes

Labeled probes were prepared from the sequences of mouse and human GT01 and mouse and human GLP-1 by an in vitro transcription method. The labeling with digoxigenin was carried out using DIG RNA Labeling Kit (Roche Diagnostics, Switzerland) and setting the total amount of a reactionmixture (plasmid DNA, 1×DIG RNA labeling Mix, 1×Transcription buffer, 1 U/ml RNasin, 2 U/ml T7 or SP6 RNA polymerase, RNase-free dH₂O) to 20 ml. This reaction solution was subjected to reaction 37°C for 2 hours before digesting the plasmid DNA using a DNase, followed by terminating the reaction employing 1 ml of 0.5 M EDTA. The synthesized cRNA probes were each ethanol precipitated and centrifuged (at 15, 000 rpm and 4°C for 15 minutes) before drying the resultant pellet, followed by dissolution in an alkaline hydrolyzing solution (40 mM NaHCO₃, 60 mM Na₂CO₃, pH 10.2) before fragmentation treatment at 60°C for 9 minutes. After the treatment, ethanol precipitation was again performed, and the precipitate was dissolved in DEPC water (Milli-Q water treated with 0.1% DEPC overnight and then heated in an autoclave at 121°C for 40 minutes for detoxication).

### (2) In situ hybridization

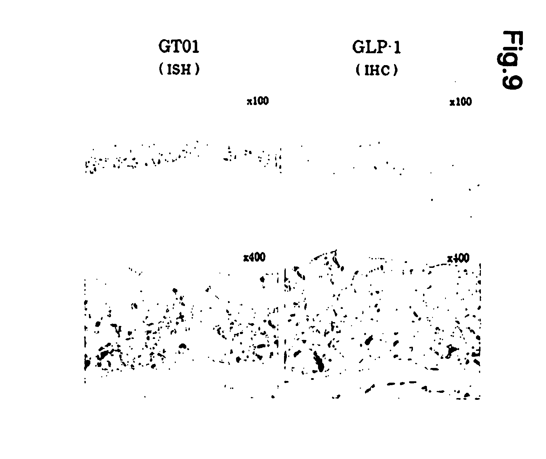

Mouse (C57BL/6, male, 8-week old) colon was perfusion fixed using a fixative (from Genostaff), and the colon was then taken and again fixed with the same fixative, followed by preparing a paraffin-embedded block. Four-µm slices were prepared from the resultant paraffin-embedded block, followed by in situ hybridization using the probes for GT01 and GLP-1. Staining was performed based on the method of Genostaff Inc. After the hybridization reaction, the probes were washed before replacement with TNE (10 mM Tris-HC1 (pH 7.6), 500 mM NaCl, 1 mM EDTA) for 10 minutes, followed by decomposing excess probes using 20 mg/ml RNase (diluted with TNE). Washing was carried out with TNE for 10 minutes, followed by washing with 2×SSC, 1×SSC, and 0.5×SSC at 55°C for 20 minutes. To detect a signal, replacement by TBS (100 mM Tris-HCl (pH 7.5), 150 mM NaCl) was performed for 5 minutes, followed by the reaction of blocking a DIG antibody at 37°C for one hour using a 1.5% blocking reagant (dilution with TBS). Washing was carried out with TBS for 5 minutes, and antibody reaction was performed at room temperature for one hour using a sheep anti-DIG antibody ((Roche Diagnostics, Switzerland) 1:500 (dilution with 1.5% blocking reagent)). The antibody was removed by washing with TBST (100 mM Tris-HCl (pH 7.5), 150 mM NaCl, 0.1% Tween 20), and replacement by NTM (100 mM Tris-HCl (pH 9.5), 100 mM NaCl, 50 mM MgCl₂) was carried out for 3 minutes. Then, color development was performed under microscopic examination using 0.34 mg/ml NBT and 0.18% BCIP (dilution with NTM), and the color development reaction was terminated by treatment with a reaction termination solution (10 mM Tris-HCl, 1 mM EDTA (pH 8.0)) for 10 minutes. After the color development, the slices were each placed on a slide glass in PBS, mounted with 90% glycerol (dilution with PBS), and subjected to microscopic examination using an optical microscope (Figure 9).

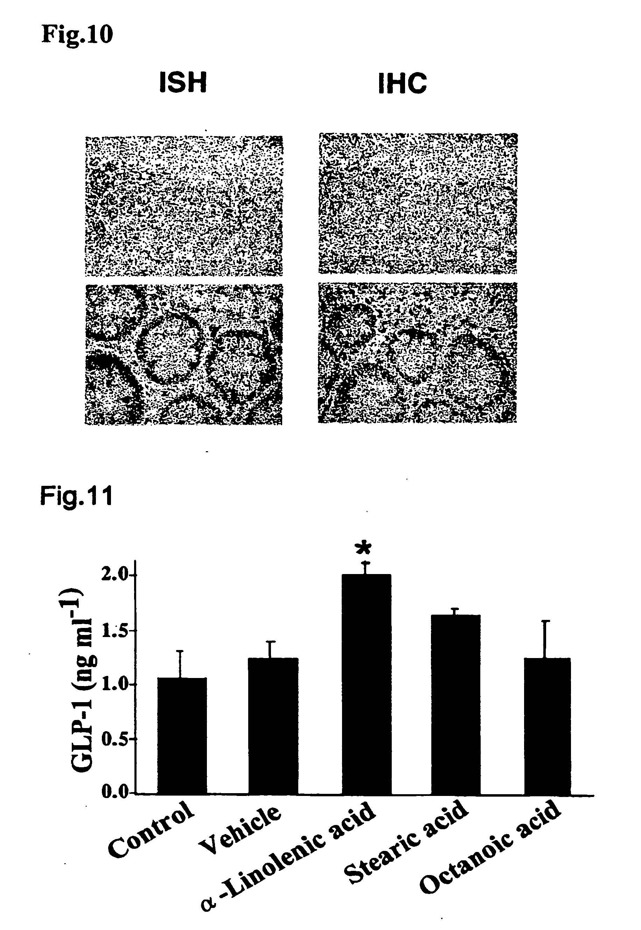

The human colon sample was also observed using the same method as that described above (Figure 10).

As a result of observation, it was shown that the sites of expression of GT01 and GLP-1 coincided closely with each other.

### Example 11

### In vivo effects of ligands for GT01 on GLP-1 release

Effects of ligands (free fatty acids) for GT01 on GLP-1 release were examined in vivo.

A 100 nmol/g of fatty acid (α-linolenic acid, stearic acid, or octanoic acid) was included in polyethyleneglycol as a vehicle, and given, through a tube, directly into the stomach of 8-week old male C57/B6 mice (Sankyo Lab) after 24-hour fasting. The mice were each anaesthetized with diethyl ether 0.5 or 2 hours after the administration of the fatty acid, followed by sampling the blood from the portal vein using a heparinized syringe. The heparizized blood was centrifuged at 4°C and 1,200xg for 20 minutes to prepare a plasma which was then subj ected to enzyme immunoassay for GLP-1 (Yanaihara Institute Inc.). All experiments were performed according to an institute-approved guideline. As a result, the secretion of GLP-1 was induced particularly when α-linolenic acid or stearic acid was given (Figure 11).

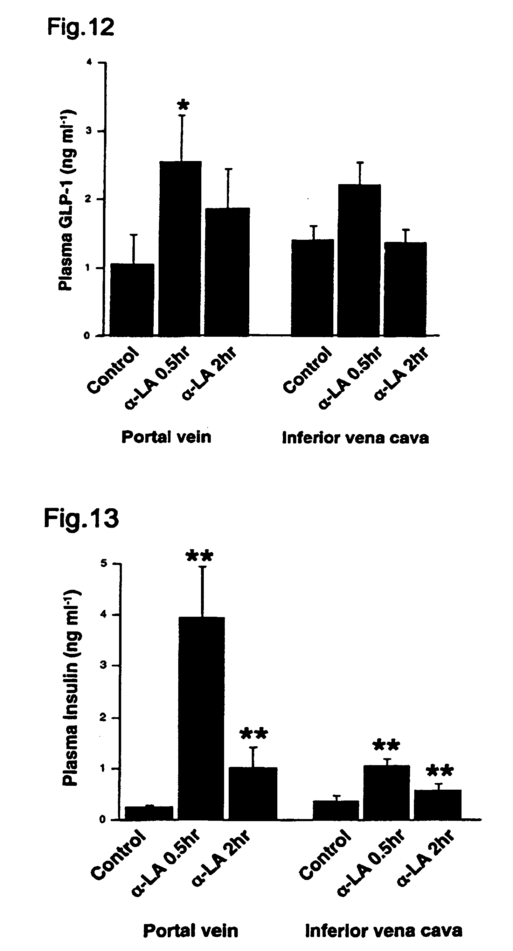

In addition, the concentration of GLP-1 in the plasma taken from the portal vein or inferior vena cava was measured using the same method (Figure 12).

### Example 12

### An in vivo effect of a ligand for GT01 on insulin release

An effect of a ligand (free fatty acid) for GT01 on insulin release was examined in vivo.

A 100 nmol/g of fatty acid (α-linolenic acid) was included in polyethyleneglycol as a vehicle, and given, through a tube, directly into the stomach of 8-week old male C57/B6 mice (Sankyo Lab) after 24-hour fasting. The mice were each anaesthetized with diethyl ether 2 hours after the administration of the fatty acid, followed by sampling the blood from the portal vein using a heparinized syringe. The heparizized blood was centrifuged at 4°C and 1,200xg for 20 minutes to prepare a plasma, in which insulin was quantitated using an enzyme immunoassay kit of insulin (Morinaga) . All experiments were performed according to an institute-approved guideline. As a result, the secretion of a higher concentration of insulin was detected in the plasma sampled from the portal vein than in that from the inferior vena cava (Figure 13).

### References

Anderson, et al., Science 256: 808-813, 1992
Ausubel, et al., Current protocols in molecular biology, John Wiley & Sons, New York, 1987
Beardshall, et al., Lancet ii 1008-1010, 1989
Brynes, A.E., Am J Clin Nutr 72: 1111-1118, 2000
Chen and Okayama, Bio Techniques, 6: 632-638, 1988
Cohen, et al., Oligodeoxynucleotides: Antisense inhibitors of gene expression, CRC Press, Boca Raton, FL, p255, 1989
Drucker, Diabetes 47: 159-169, 1998
Drucker, Endocrinology 142: 521-527, 2001
Elroy-Stein and Moss, Proc. Natl. Acad. Sci. USA 87: 6743-6747, 1990
Eppstein, et al., Proc. Natl. Acad. Sci. USA 82: 3688-92, 1985
Fredriksson, et al., FEBS 554: 381-388, 2003
Flint, et al., J Clin Invest 101: 515-520, 1998
Gennaro, et al.: The science and practice of pharmacy. Lippincott, Williams & Wilkins, Philadelphia, PA, 2000
Guimbaud, et al., Pancreas 14: 76-82, 1997
Goding, et al., Academic Press, San Diego, p492, 1996
Harlow and Lane, Antibodies: A laboratory manual, Cold Spring Harbor Laboratory Press, Cold Spring Harbor, p726, 1988
Harlow and Lane, Using antibodies: A laboratory manual, Cold Spring Harbor Laboratory Press, Cold Spring Harbor, New York, 1999
Cuche, G. , et al., AmJ Physiol Gastrointest Liver Physiol, 279: G925-930, 2000
Higham, et al., Gut 41: 24-32, 1997
Hopman, et al., Gastroenterology 89: 1242-1247, 1985
Hwang, et al., Proc Natl Acad Sci USA, 77: 4030-4, 1980
Isaacs, et al., Digestive Diseases and Sciences 32: 451-480, 1987
Jones, et al., Nature, 321: 522-5, 1986
Kozbor, et al., J Immunol, 133: 3001-5, 1984
Liddle, Annual Review of Physiol 59: 221-242, 1997
Liddle, et al., Journal of Clinical Investigation 72: 992-996, 1986
Lopata, et al., Nucleic Acids Research, 12: 5707, 1984
Martin and Papahadjopoulos, J Biol Chem, 257: 286-8, 1982
Milstein, et al., Nature, 305: 537-40, 1983
Morrison, et al., Genetically engineered antibody molecules and their application, Ann NY Acad Sci, 507: 187-98, 1987
Okano, et al., J Neurochem, 56: 560-7, 1991
Presta, et al., Curr Opin Biotechnol, 3: 394-8, 1992
Reimer, R.A., et al., Endocrinology 142 : 4522-4528, 2001
Riechmann, et al., Nature, 332: 323-7, 1988
Rocca, A.S., et al., Endocrinology 142: 1148-1155, 2001
Sambrook, J. Molecularcloning: alaboratorymanual, Cold Spring Harbor Laboratory, Cold Spring Harbor, 1989
Schade, et al., The production of avian (egg yolk) antibodies: IgY, The report and recommendations of ECVAM workshop, Alternatives to Laboratory Animals (ATLA), 24: 925-934, 1996
Sidhu, et al., J. Physiol, 528.1: 165-176, 2000
Smith and Gibbs, J. Annals of the New York Academy of Science 713: 236-241, 1994.
Thorens and Waeber, Diabetes 42: 1219-1225, 1993
Tonkinson, et al., Cancer Investigation, 14(1): 54-65, 1996
Turton, et al., Nature 379: 69-72, 1996
Verhoeyen, et al., Science, 239: 1534-6, 1988

### Industrial Applicability

The present invention aids in the development of a pharmaceutical preparation for the lowering of elevated blood sugar level in diabetes or the like and for the prevention of the elevation, and particularly also enables the development of a pharmaceutical preparation suitable for the treatment of patients whose obese symptoms are marked.

### Brief Description of the Drawings

Figure 1 is a scheme of a 7-pass transmembrane receptor;
Figure 2 is an image showing the tissue specificity of a mouse GT01 gene expression. GAPDH (glyceraldehydes-3-phosphate dehydrogenase) was used as a control for comparing the expression;
Figure 3 is a fluorescence microscopic image showing that, as a result of the binding of a ligand to a GT01 polypeptide, the polypeptide migrates into the cell together with the ligand;
Figure 4 is the results of determining the increase in intracellular calcium concentration induced by a free fatty acid by detecting fluorescence intensity at 510 to 570 nm for excitation light of 488 nm using FLIPR (Fluorometric Imaging Plate Reader, Molecular Devices);
Figure 5 is a diagram showing pEC₅₀s for increases in the concentration of Ca²⁺ in an HEK cell when various free fatty acids were added;
Figure 6 is a set of graphs showing an ef f ect of anantisense for human GT01 on the increase in intracellular calcium concentration induced by α-linolenic acid;
Figure 7 is a graph showing the free fatty acid concentration dependency of the CCK release induced by various free fatty acids;
Figure 8 is a graph showing the amounts of the GLP-1 release (ordinate) induced by various fatty acids (α-linolenic acid, docosahexaenoic acid (DHA), palmitoleic acid, oleic acid, stearic acid, octanoic acid, and α-linolenic acid-methyl ester). The fatty acids are each used in concentrations of 10 µM, 3 µM, and 100 µM (abscissa);
Figure 9 is the results of in situ hybridization regarding GT01 and GLP-1 in mouse colon;
Figure 10 is the results of in situ hybridization regarding GT01 and GLP-1 in human colon;
Figure 11 is a graph showing the variation of GLP-1 concentration in mouse portal vein by giving various fatty acids (α-linolenic acid, stearic acid, and octanoic acid) to the mice. For negative controls, the determination of concentration was carried out when neither fatty acid nor vehicle were given (control) and when only vehicle was given (vehicle);
Figure 12 is a graph showing the concentration of GLP-1 in mouse portal vein or inferior vena cava 0.5 hr or 2 hrs after giving α-linolenic acid (α-LA) to the mice. Fornegative controls, the determination of concentration was carried out when neither fatty acid nor vehicle were given (control) and when only vehicle was given (vehicle); and
Figure 13 is a graph showing the concentration of insulin in mouse portal vein or inferior vena cava 0.5 hr or 2 hrs after giving α-linolenic acid (α-LA) to the mice. For negative controls, the determination of concentration was carried out when neither fatty acid nor vehicle was given (control) and when only vehicle was given (vehicle).

## Claims

1. Use of linolenic acid for preparation of a pharmaceutical composition for lowering the high blood sugar level associated with diabetes through inducing insulin secretion.

2. Use of linolenic acid for the preparation of a pharmaceutical composition for treating eating disorders through promoting GLP-1 secretion.

3. Use of linolenic acid for the preparation of a pharmaceutical composition for treating obesity through promoting GLP-1 secretion.

4. Use of linolenic acid for the preparation of a pharmaceutical composition for treating overeating through promoting GLP-1 secretion.

5. Use of Linolenic acid for the preparation of a dietary supplement composition for rational dieting or suppressing overeating through promoting GLP-1 secretion.

6. Use of linolenic acid for manufacture of a medicament for promoting GLP-1 secretion from a cell.

7. Use of linolenic acid for manufacture of a medicament for promoting CCK secretion from a cell.

8. Use of linolenic acid for manufacture of a medicament for inducing insulin secretion.

9. Use according to any one of claims 1 to 8, wherein the linolenic acid is α-linolenic acid.

10. Use of docosahexaenoic acid for manufacture of a medicament for promoting GLP-1 secretion.

11. Linolenic acid for use for the treatment of a patient by lowering the high blood sugar level associated with diabetes through inducing insulin secretion.

12. Linolenic acid for use for the treatment of eating disorders through promoting GLP-1 secretion.

13. Linolenic acid for use for the treatment of obesity through promoting GLP-1 secretion.

14. Linolenic acid for use for the treatment of overeating through promoting GLP-1 secretion.

15. Linolenic acid for use for the treatment of overeating as a dietary supplement composition for rational dieting or suppressing overeating through promoting GLP-1 secretion.

16. Linolenic acid for use for the treatment of a patient by promoting GLP-1 secretion from a cell.

17. Linolenic acid for use for the treatment of a patient by promoting CCK secretion from a cell.

18. Linolenic acid for use for the treatment of a patient by inducing insulin secretion.

19. Linolenic acid for use for a treatment according to any one of claims 11 to 18, wherein the linolenic acid is α-linolenic acid.

20. Docosahexaenoic acid for use for the treatment of a patient by promoting GLP-1 secretion.

## Patentansprüche

1. Verwendung von Linolensäure in der Zubereitung einer pharmazeutischen Zusammensetzung zur Senkung des hohen Blutzuckerwertes der mit Diabetes zusammenhängt, durch Einleiten einer Insulinsekretion.

2. Verwendung von Linolensäure in der Zubereitung einer pharmazeutischen Zusammensetzung zur Behandlung von Essstörungen durch Förderung der GLP-1-Sekrektion.

3. Verwendung von Linolensäure in der Zubereitung einer pharmazeutischen Zusammensetzung zur Behandlung von Fettleibigkeit durch Förderung der GLP-1-Sekrektion.

4. Verwendung von Linolensäure in der Zubereitung einer pharmazeutischen Zusammensetzung zur Behandlung von Überernährung durch Förderung der GLP-1-Sekrektion.

5. Verwendung von Linolensäure in der Zubereitung einer Nahrungsergänzungszusammensetzung für eine vernünftige Ernährung oder Einschränkung einer Überernährung durch Förderung der GLP-1-Sekrektion.

6. Verwendung von Linolensäure in der Herstellung eines Medikaments zur Förderung der GLP-1-Sekretion von einer Zelle.

7. Verwendung von Linolensäure in der Herstellung eines Medikaments zur Förderung der CCK-Sekretion von einer Zelle.

8. Verwendung von Linolensäure in der Herstellung eines Medikaments zur Einleitung einer Insulinsekretion.

9. Verwendung nach einem der Ansprüche 1 bis 8, wobei die Linolensäure α-Linolensäure ist.

10. Verwendung von Docosahexaensäure in der Herstellung eines Medikaments zur Förderung der GLP-1-Sekretion.

11. Linolensäure zur Verwendung in der Behandlung eines Patienten durch Senken des hohen Blutzuckerwertes der mit Diabetes zusammenhängt, durch Einleiten einer Insulinsekretion.

12. Linolensäure zur Verwendung in der Behandlung von Essstörungen durch Förderung der GLP-1-Sekrektion.

13. Linolensäure zur Verwendung in der Behandlung von Fettleibigkeit durch Förderung der GLP-1-Sekrektion.

14. Linolensäure zur Verwendung in der Behandlung von Überernährung durch Förderung der GLP-1-Sekrektion.

15. Linolensäure zur Verwendung in der Behandlung von Überernährung als Nahrungsergänzungszusammensetzung für eine vernünftige Ernährung oder Einschränkung einer Überernährung durch Förderung der GLP-1-Sekrektion.

16. Linolensäure zur Verwendung in der Behandlung eines Patienten durch Förderung der GLP-1-Sekretion von einer Zelle.

17. Linolensäure zur Verwendung in der Behandlung eines Patienten durch Förderung der CCK-Sekretion von einer Zelle.

18. Linolensäure zur Verwendung in der Behandlung eines Patienten durch Einleiten einer Insulinsekretion.

19. Linolensäure zur Verwendung in der Behandlung nach einem der Ansprüche 11 bis 18, wobei die Linolensäure α-Linolensäure ist.

20. Docosahexaensäure zur Verwendung in der Behandlung eines Patienten durch Förderung der GLP-1-Sekrektion.

## Revendications

1. Utilisation d'acide linolénique pour la préparation d'une composition pharmaceutique destinée à réduire le niveau élevé de glycémie associé au diabète par induction de la sécrétion d'insuline.

2. Utilisation d'acide linolénique pour la préparation d'une composition pharmaceutique destinée au traitement de troubles de l'alimentation par promotion de la sécrétion de GLP-1.

3. Utilisation d'acide linolénique pour la préparation d'une composition pharmaceutique destinée au traitement de l'obésité par promotion de la sécrétion de GLP-1.

4. Utilisation d'acide linolénique pour la préparation d'une composition pharmaceutique destinée au traitement de l'hyperphagie par promotion de la sécrétion de GLP-1.

5. Utilisation d'acide linolénique pour la préparation d'une composition de supplément alimentaire pour un régime rationnel ou pour la suppression de l'hyperphagie par promotion de la sécrétion de GLP-1.

6. Utilisation d'acide linolénique pour la fabrication d'un médicament destiné à promouvoir la sécrétion de GLP-1 d'une cellule.

7. Utilisation d'acide linolénique pour la fabrication d'un médicament destiné à promouvoir la sécrétion de CCK d'une cellule.

8. Utilisation d'acide linolénique pour la fabrication d'un médicament destiné à induire la sécrétion d'insuline.

9. Utilisation selon l'une quelconque des revendications 1 à 8, dans laquelle l'acide linolénique est l'acide a-linolénique.

10. Utilisation d'acide docosahexaénoïque pour la fabrication d' un médicament destiné à promouvoir la sécrétion de GLP-1.

11. Acide linolénique destiné à être utilisé pour le traitement d'un patient par réduction du niveau élevé de glycémie associé au diabète par induction de la sécrétion d'insuline.

12. Acide linolénique destiné à être utilisé pour le traitement de troubles de l'alimentation par promotion de la sécrétion de GLP-1.

13. Acide linolénique destiné à être utilisé pour le traitement de l'obésité par promotion de la sécrétion de GLP-1.

14. Acide linolénique destiné à être utilisé pour le traitement de l'hyperphagie par promotion de la sécrétion de GLP-1.

15. Acide linolénique destiné à être utilisé pour le traitement de l'hyperphagie en tant que composition de supplément alimentaire pour un régime rationnel ou pour la suppression de l'hyperphagie par promotion de la sécrétion de GLP-1.

16. Acide linolénique destiné à être utilisé pour le traitement d'un patient par promotion de la sécrétion de GLP-1 d'une cellule.

17. Acide linolénique destiné à être utilisé pour le traitement d'un patient par promotion de la sécrétion de CCK d'une cellule.

18. Acide linolénique destiné à être utilisé pour le traitement d'un patient par induction de la sécrétion d'insuline.

19. Acide linolénique destiné à être utilisé pour un traitement selon l'une quelconque des revendications 11 à 18, l'acide linolénique étant de l'acide a-linolénique.

20. Acide docosahexaénoïque destiné à être utilisé pour le traitement d'un patient par promotion de la sécrétion de GLP-1.
